# EUROPEAN PATENT APPLICATION

(11) **EP 4 290 347 A1**
(43) Date of publication of application: **13.12.2023**
(21) Application number: 21924813.5
(22) Date of filing: 07.12.2021
(51) Int. Cl.: G06F 3/04815, A61B 5/107, A61B 5/11, G06F 3/01

(54) **INFORMATION PROCESSING METHOD, INFORMATION PROCESSING DEVICE, AND PROGRAM**

(30) Priority: 02.02.2021 JP 2021014952
(71) Applicant: Sony Group Corporation, Tokyo 108-0075 (JP)
(72) Inventor: ISHII, Tamotsu, Tokyo 108-0075 (JP); SATO, Tetsuro, Tokyo 108-0075 (JP); FUKUMOTO, Yasutaka, Tokyo 108-0075 (JP)
(74) Representative: MFG Patentanwälte Meyer-Wildhagen Meggle-Freund Gerhard PartG mbB
(86) International application number: PCT/JP2021/044934
(87) International publication number: WO 2022/168428

(57) **Abstract**

[Object]

To deal with the generation of inexact posture information.

[Solving Means]

An information processing method includes acquiring posture information that indicates a posture of a moving body, extracting a feature amount from the posture information acquired at one time point or at multiple time points, determining whether or not the extracted feature amount is included within a definition range in a feature amount space, and acquiring, as a use feature amount, a feature amount that is included within the definition range, when the extracted feature amount is determined not to be included within the definition range, and generating data that indicates a posture or a motion, by using the use feature amount.

## Description

### [Technical Field]

The present disclosure relates to an information processing method, an information processing device, and a program.

### [Background Art]

In recent years, motion capture technologies for acquiring motion information which indicates a motion of a user have actively been developed. The acquired motion information has been used to improve forms in sports or used for VR (Virtual Reality) or AR (Augmented Reality) applications, for example. In addition, the acquired motion information is also used to generate an avatar image simulating a motion of a user. The generated avatar image is then distributed.

It is to be noted that an optical system using markers, a sensor system using an acceleration sensor or the like, and a camera system for analyzing images are known as systems for implementing the motion capture technology. For example, PTL 1 discloses a motion capture technology that is implemented by the sensor system. It is to be noted that the motion information is time series data including a series of pieces of posture information indicating the posture of a user at a certain time.

### [Citation List]

### [Patent Literature]

[PTL 1]
PCT Patent Publication No. WO2019/203188

### [Summary]

### [Technical Problem]

However, posture information that is acquired according to the motion capture technology is inexact in some cases. For example, in the sensor system, inexact posture information is acquired in a case where a sensor worn on a user slips down or is displaced. Also in the optical system or the camera system, the accuracy of posture information can be degraded due to displacement of a camera or occurrence of a drift. As a result, there is a concern that, in a case where the motion capture technology is applied to generation of avatar images, for example, an avatar image may be generated on the basis of an inexact posture or an inexact motion.

Therefore, the present disclosure proposes a new and improved information processing method, information processing device, and program which are capable of dealing with generation of inexact posture information.

### [Solution to Problem]

According to the present disclosure, there is provided an information processing method including acquiring posture information that indicates a posture of a moving body, extracting a feature amount from the posture information acquired at one time point or at multiple time points, determining whether or not the extracted feature amount is included within a definition range in a feature amount space, and acquiring, as a use feature amount, a feature amount that is included within the definition range, when the extracted feature amount is determined not to be included within the definition range, and generating data that indicates a posture or a motion, by using the use feature amount.

In addition, according to the present disclosure, there is provided an information processing device including a posture information acquisition section that acquires posture information that indicates a posture of a moving body, a feature amount extraction section that extracts a feature amount from the posture information acquired at one time point or at multiple time points, a determination section that determines whether or not an extracted feature amount that is the feature amount extracted by the feature amount extraction section is included within a definition range in a feature amount space, and a data generation section that generates data that indicates a posture or a motion having a feature amount included within the definition range, when the determination section determines that the extracted feature amount is not included within the definition range.

Moreover, according to the present disclosure, there is provided a program for causing a computer to function as a posture information acquisition section that acquires posture information that indicates a posture of a moving body, a feature amount extraction section that extracts a feature amount from the posture information acquired at one time point or at multiple time points, a determination section that determines whether or not an extracted feature amount that is the feature amount extracted by the feature amount extraction section is included within a definition range in a feature amount space, and a data generation section that generates data that indicates a posture or a motion having a feature amount included within the definition range, when the determination section determines that the extracted feature amount is not included within the definition range.

### [Brief Description of Drawings]

[FIG. 1]

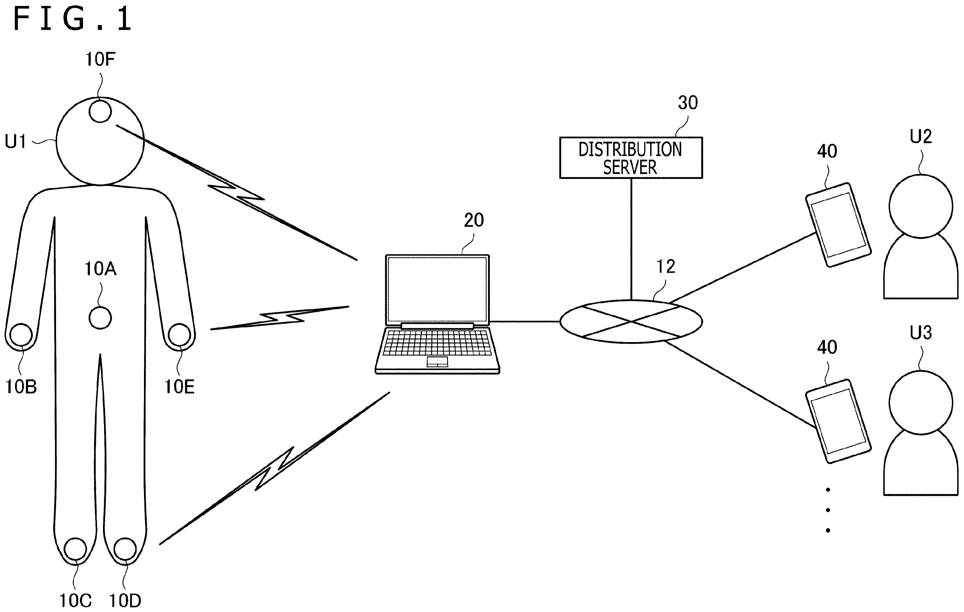

   FIG. 1 is an explanatory diagram depicting an information processing system according to one embodiment of the present disclosure.
[FIG. 2]

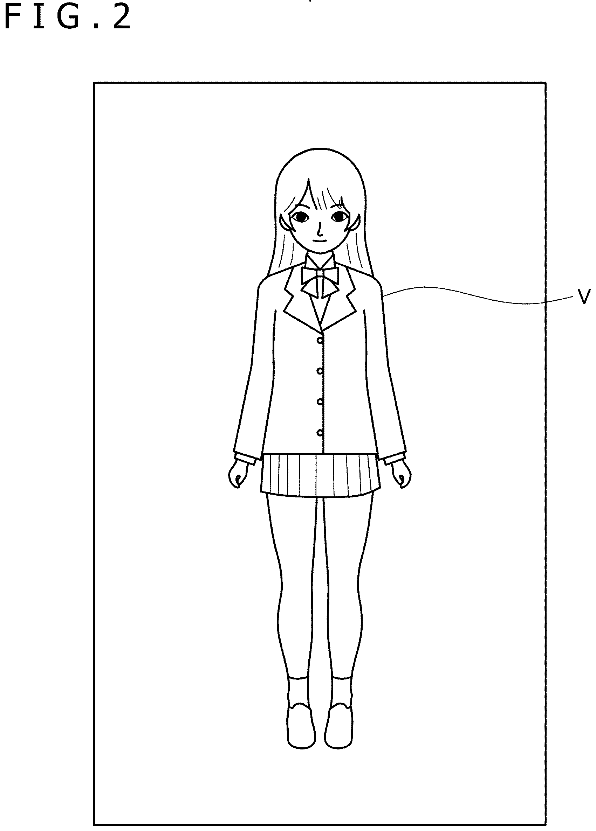

   FIG. 2 is an explanatory diagram depicting a specific example of an avatar image V that is displayed on a viewing user terminal 40.
[FIG. 3]

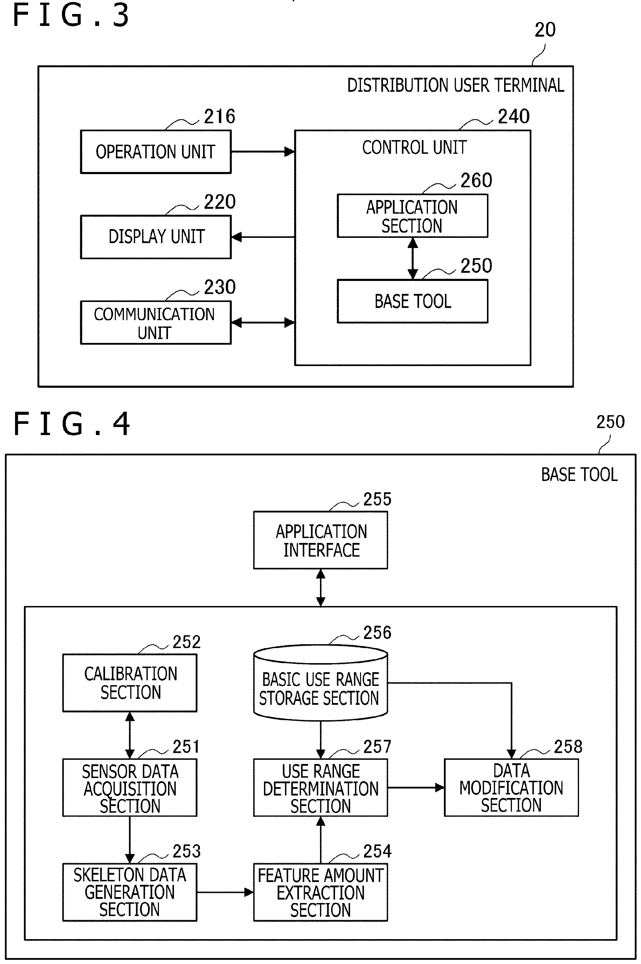

   FIG. 3 is an explanatory diagram depicting a configuration of a distribution user terminal 20 according to the one embodiment of the present disclosure.
[FIG. 4]
   FIG. 4 is an explanatory diagram depicting functions of a base tool 250.
[FIG. 5]

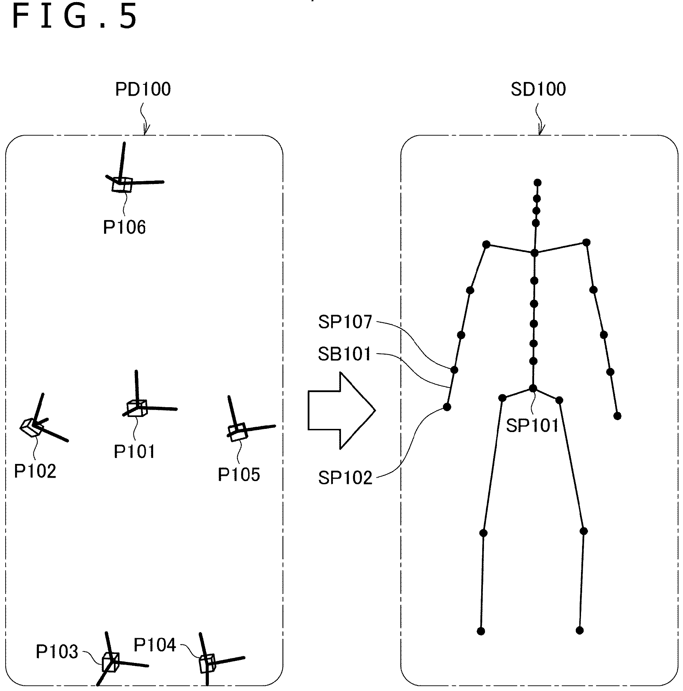

   FIG. 5 is an explanatory diagram depicting a specific example of generating raw skeleton data.
[FIG. 6]

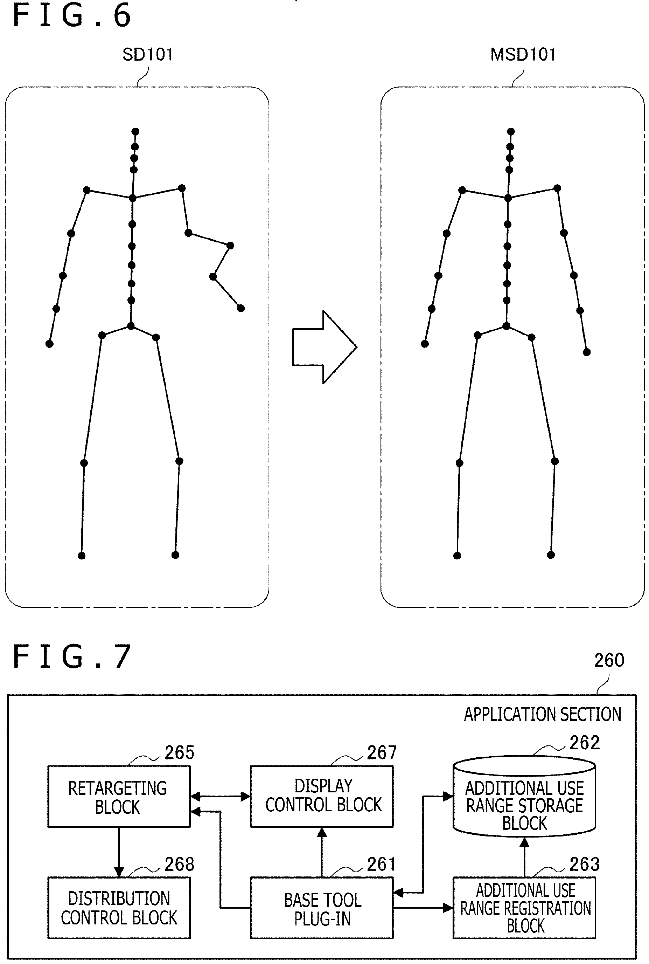

   FIG. 6 is an explanatory diagram depicting a specific example of modified skeleton data that is generated by a data modification section 258.
[FIG. 7]
   FIG. 7 is an explanatory diagram depicting functions of an application section 260.
[FIG. 8]

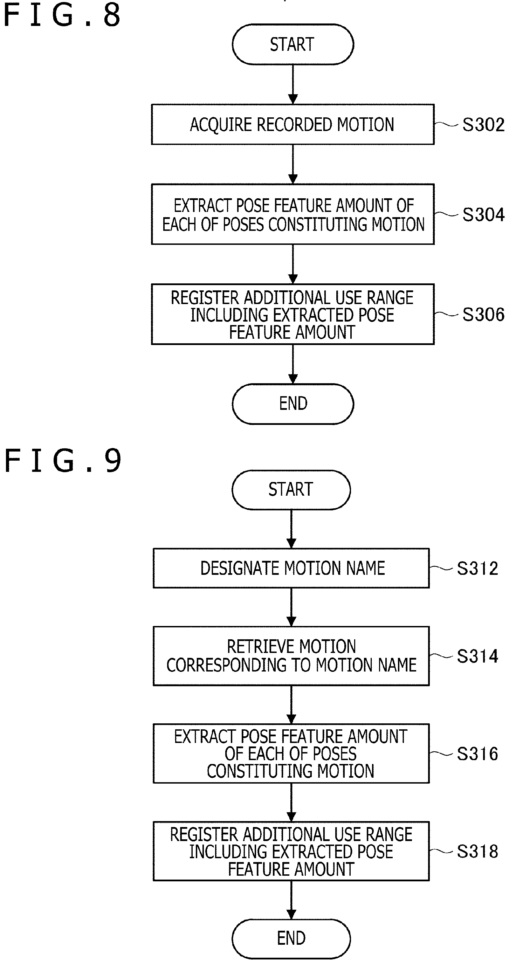

   FIG. 8 is a flowchart of a first example of registering an additional use range.
[FIG. 9]
   FIG. 9 is a flowchart of a second example of registering the additional use range.
[FIG. 10]

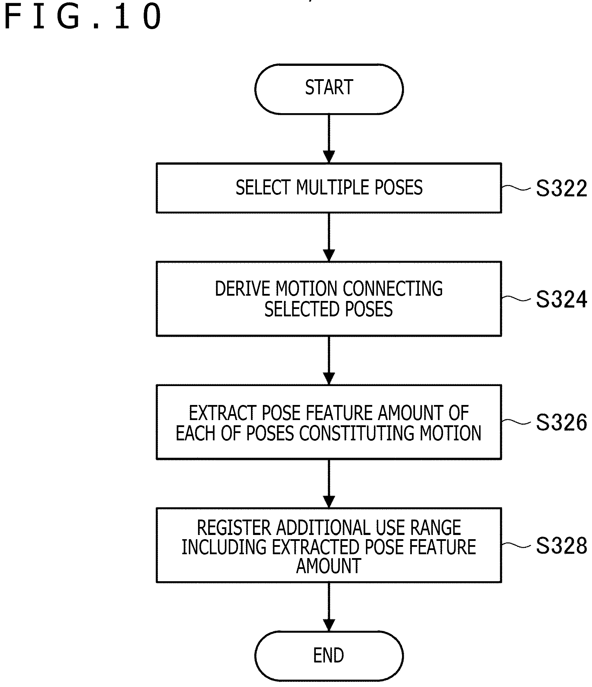

   FIG. 10 is a flowchart of a third example of registering the additional use range.
[FIG. 11]

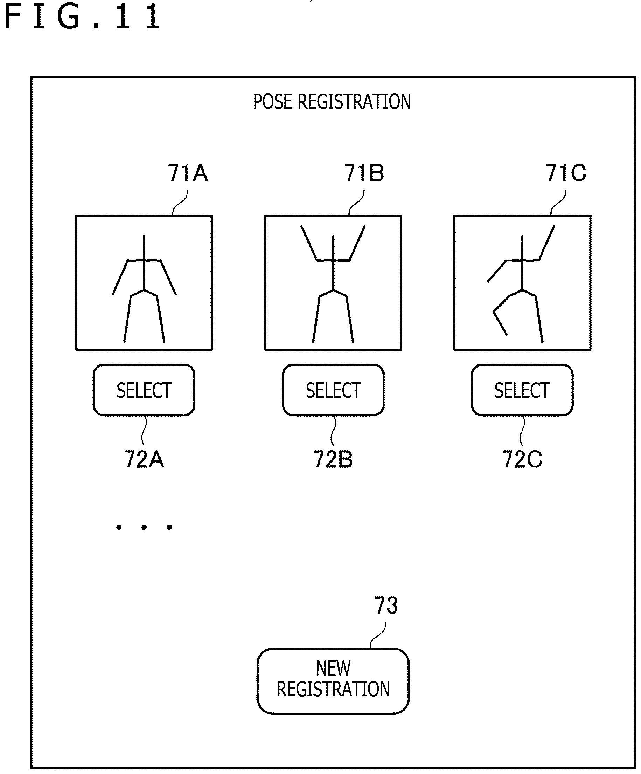

   FIG. 11 is an explanatory diagram depicting a specific example of a pose selection screen.
[FIG. 12]

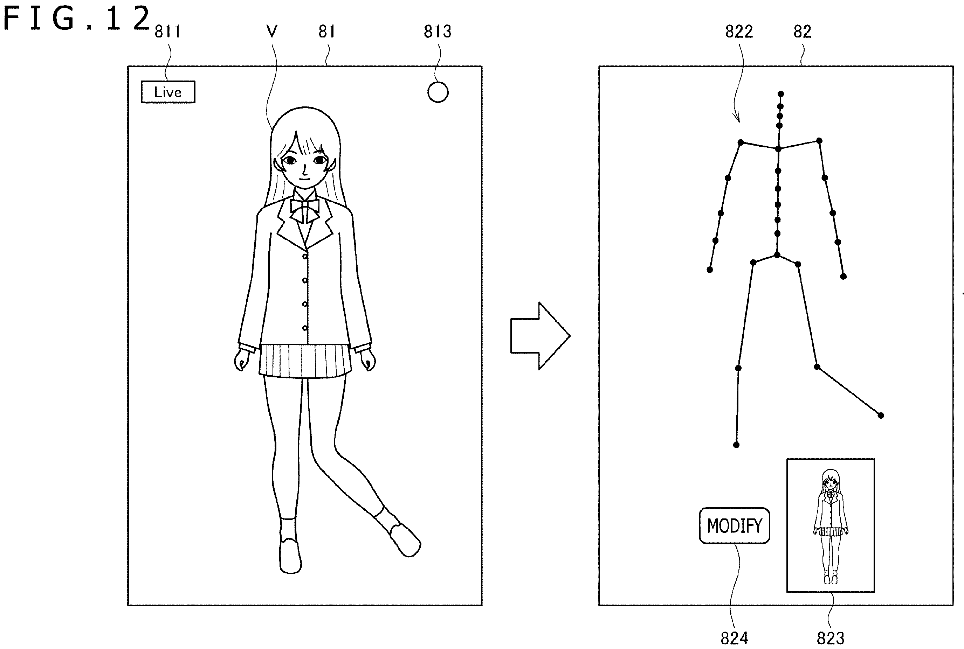

   FIG. 12 is an explanatory diagram depicting one example of a display screen designed for a distribution user.
[FIG. 13]

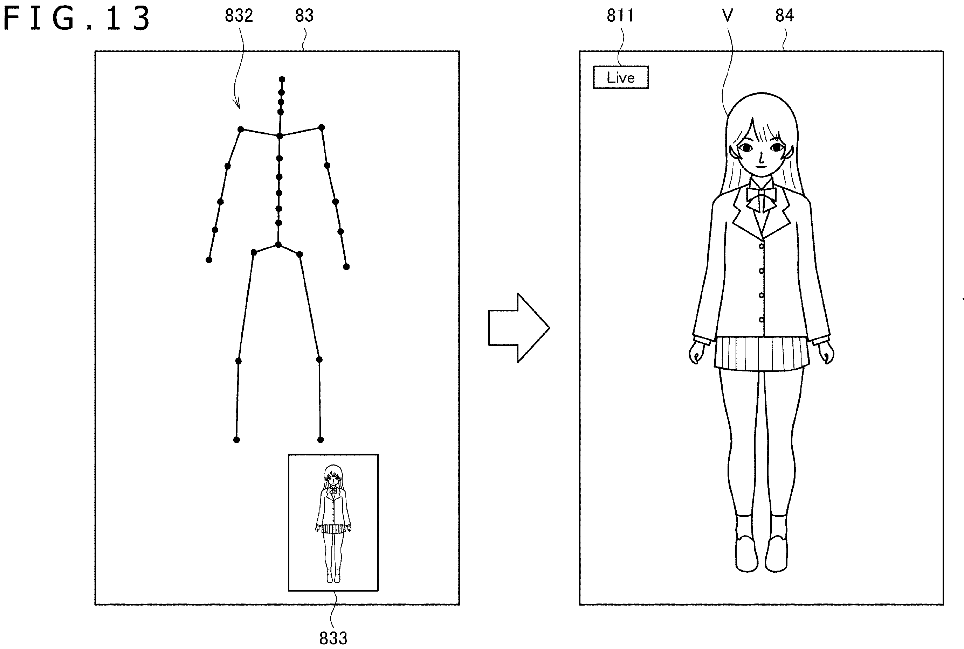

   FIG. 13 is an explanatory diagram depicting one example of a display screen designed for a distribution user.
[FIG. 14]

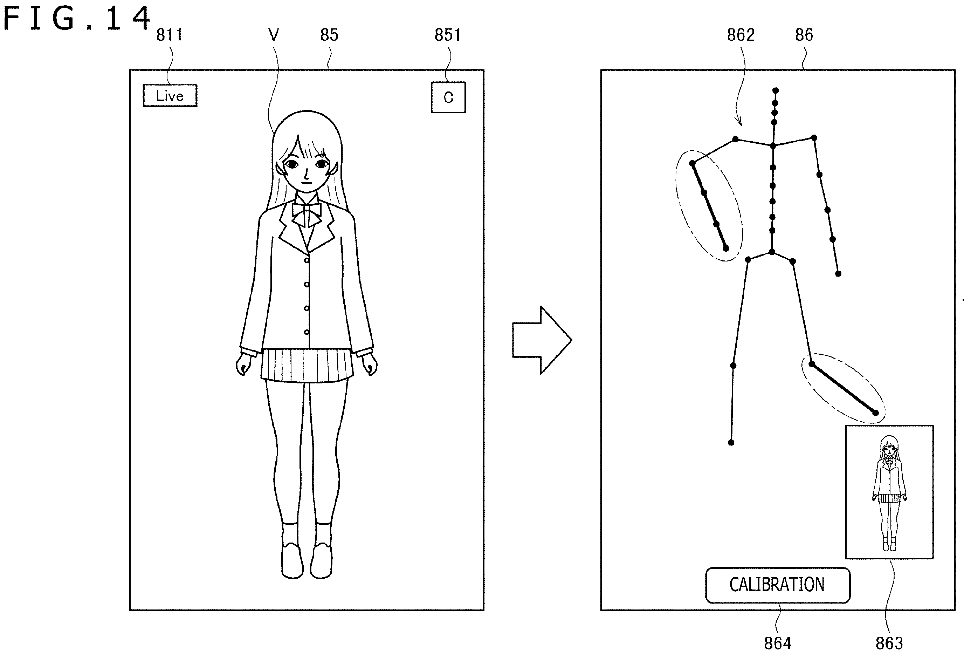

   FIG. 14 is an explanatory diagram depicting a specific example of a distribution confirmation screen 85 including a calibration icon.
[FIG. 15]

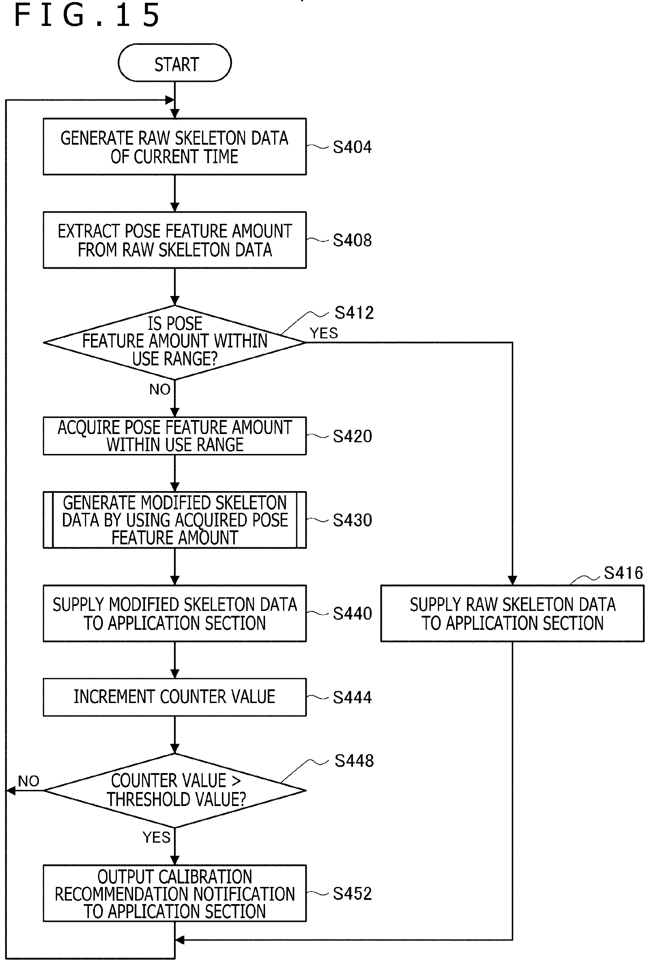

   FIG. 15 is a flowchart of the operation of the base tool 250.
[FIG. 16]

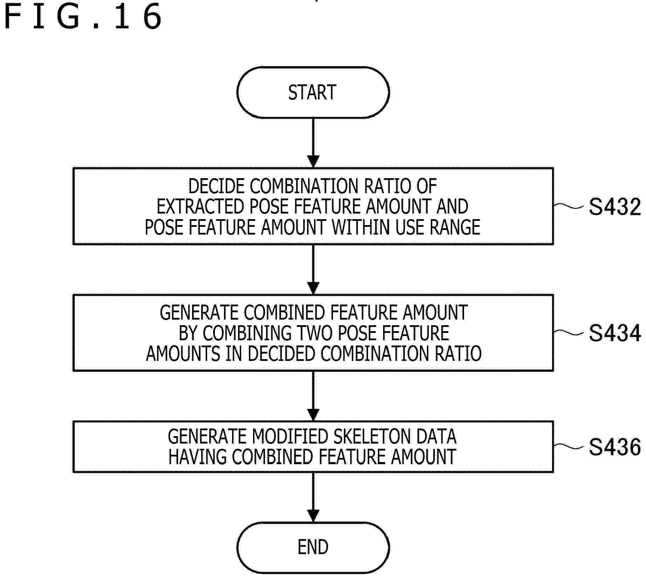

   FIG. 16 is a flowchart of a specific example of a method of generating modified skeleton data.
[FIG. 17]

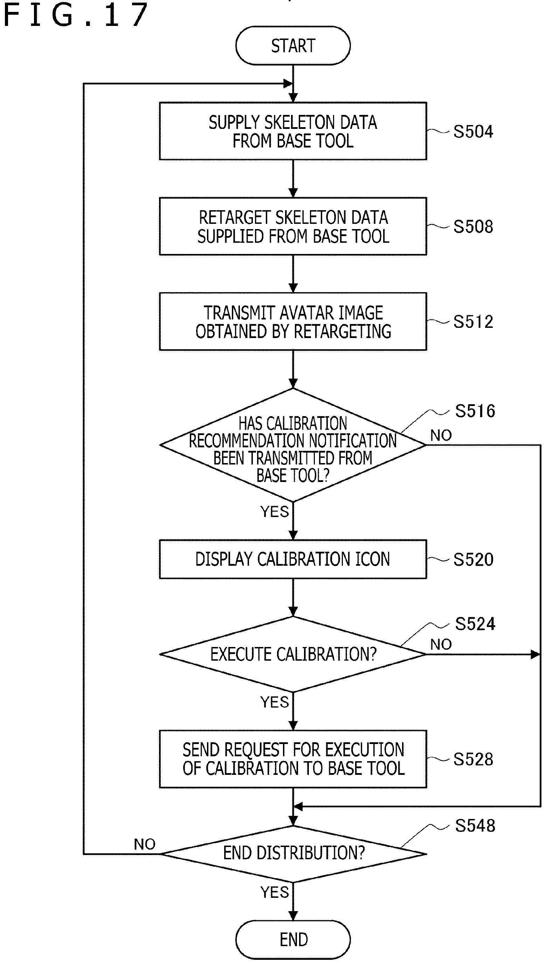

   FIG. 17 is a flowchart of the operation of the application section 260.
[FIG. 18]

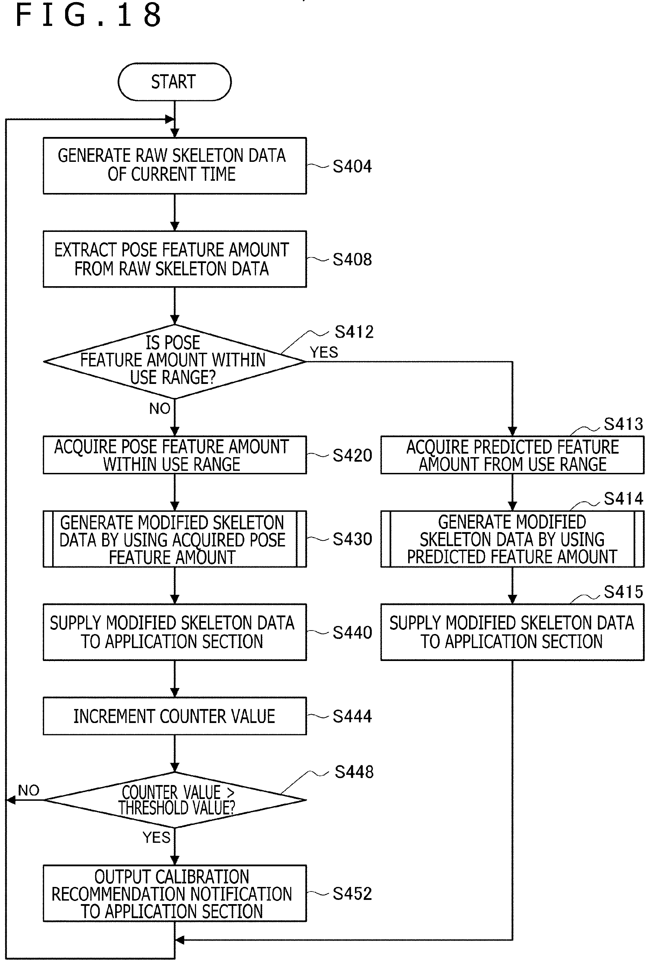

   FIG. 18 is a flowchart of a first modification of the operation of the base tool 250.
[FIG. 19]

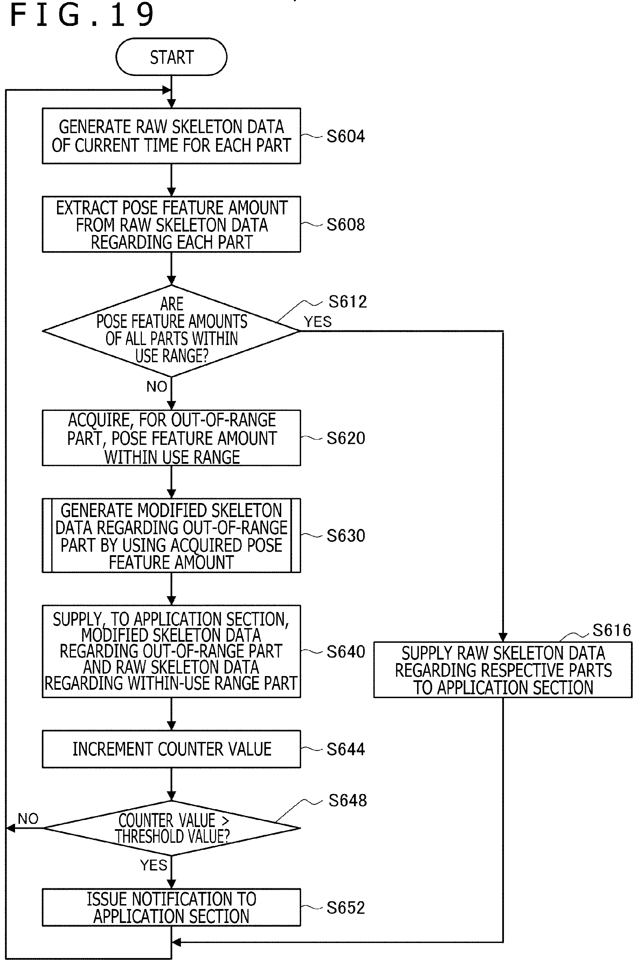

   FIG. 19 is a flowchart of a second modification of the operation of the base tool 250.
[FIG. 20]

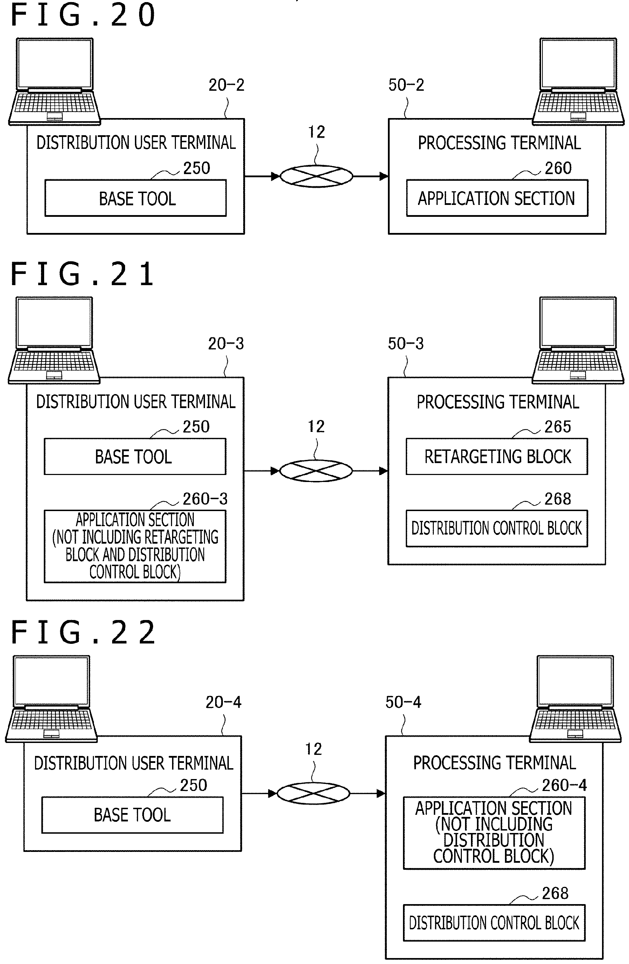

   FIG. 20 is an explanatory diagram depicting a second configuration example of the information processing system.
[FIG. 21]
   FIG. 21 is an explanatory diagram depicting a third configuration example of the information processing system.
[FIG. 22]
   FIG. 22 is an explanatory diagram depicting a fourth configuration example of the information processing system.
[FIG. 23]

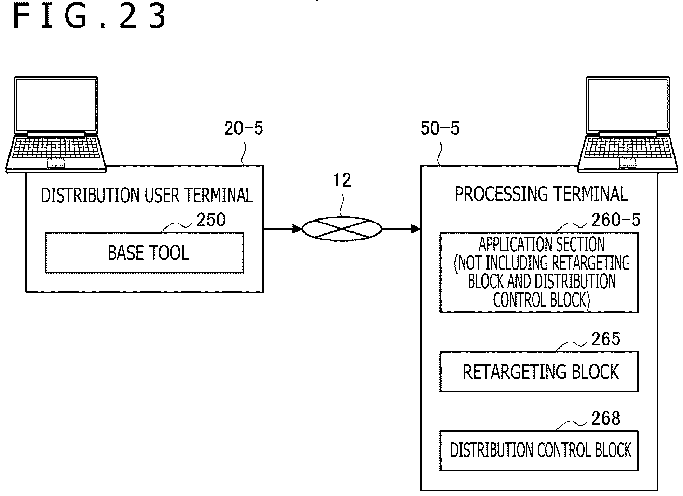

   FIG. 23 is an explanatory diagram depicting a fifth configuration example of the information processing system.
[FIG. 24]

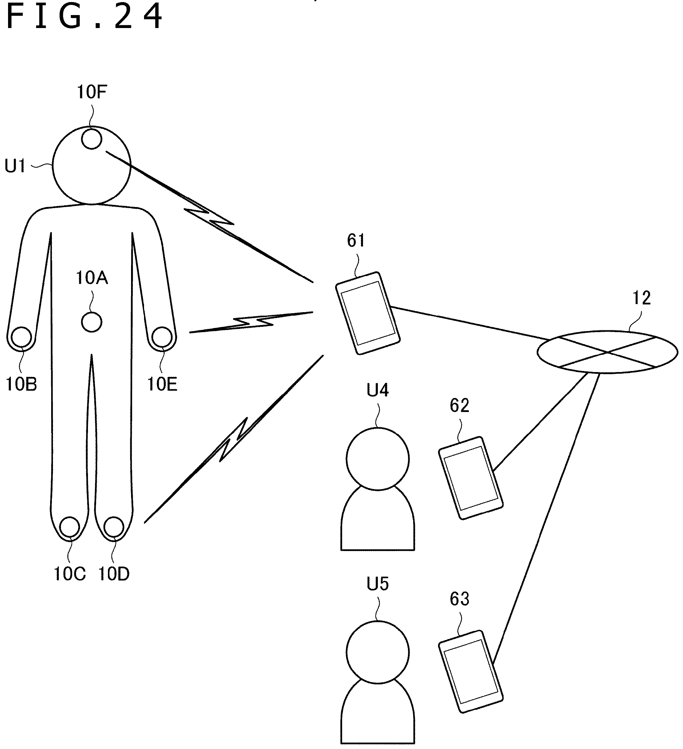

   FIG. 24 is an explanatory diagram depicting a sixth configuration example of the information processing system.
[FIG. 25]

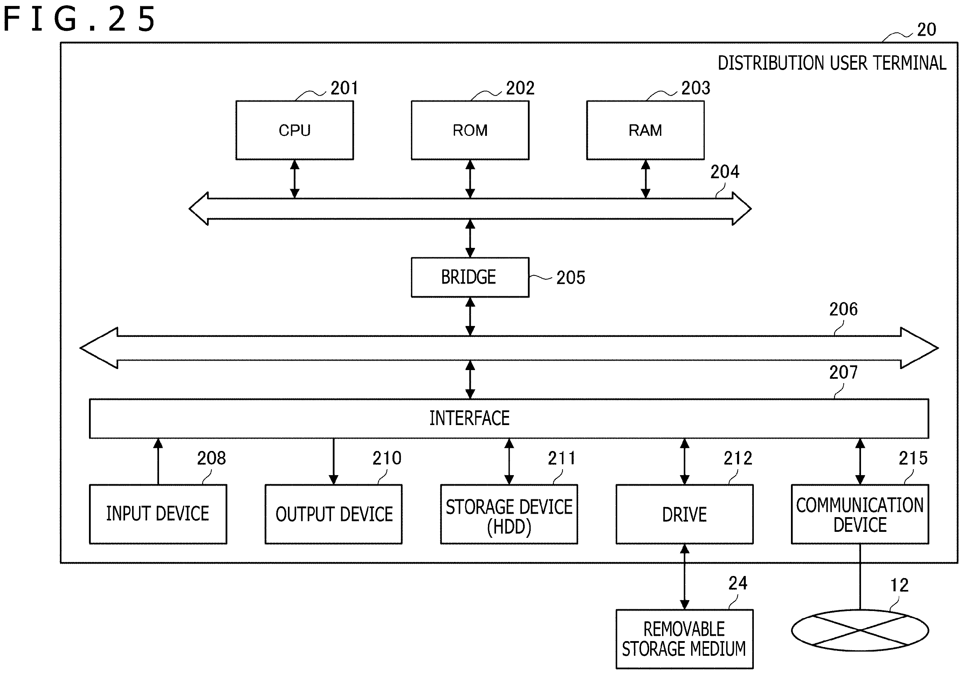

   FIG. 25 is a block diagram depicting a hardware configuration of the distribution user terminal 20.

### [Description of Embodiment]

Hereinafter, a preferable embodiment of the present disclosure will be explained in detail with reference to the attached drawings. It is to be noted that components having substantially the same functional configuration are denoted by the same reference sign throughout the present description and the drawings, and a redundant explanation thereof will be omitted.

The "description of embodiment" will be given in the following order.
1. General description of information processing system
2. Configuration of distribution user terminal
   2-1. Overall configuration
   2-2. Functions of base tool
   2-3. Functions of application section
3. Operation
   3-1. Operation of base tool
   3-2. Operation of application section
4. Summery
5. Modifications
   4-1. First modification
   4-2. Second modification
   4-3. Other modifications
6. Other configuration examples of information processing system
   5-1. Second configuration example
   5-2. Third configuration example
   5-3. Fourth configuration example
   5-4. Fifth configuration example
   5-5. Sixth configuration example
7. Hardware configuration
8. Supplemental remarks

### <<1. General description of information processing system>>

In order to visualize information regarding a motion of a moving body such as a person or an animal, skeleton data that is expressed by a skeleton structure is used. The skeleton structure indicates the structure of a body, for example. The skeleton data includes information regarding parts and bones which are line segments connecting the parts. It is to be noted that each of the parts in the skeleton structure corresponds to an end part or a joint part of the body, for example. In addition, the bones in the skeleton structure can correspond to bones of a person, for example, but the positions and number of the bones do not need to match with those of an actual person skeletal structure.

The positions of the parts in the skeleton data can be acquired according to a variety of motion capture technologies. For example, there have been available a camera system technology of acquiring the positions of markers attached to the respective parts of the body, with the use of an external camera or the like, and a sensor system technology of acquiring positional information regarding a motion sensor worn on a part of the body, on the basis of sensor data acquired by the motion sensor.

In addition, such skeleton data is used for various purposes. For example, time-series skeleton data is used to improve forms in sports or used for VR (Virtual Reality) or AR (Augmented Reality) applications, for example. Moreover, time-series skeleton data is also used to generate an avatar image that simulates a motion of a user. The generated avatar image is then distributed.

Hereinafter, a configuration example of an information processing system according to one embodiment of the present disclosure will be described. The information processing system according to the one embodiment generates skeleton data by using a motion sensor and distributes an avatar image based on the skeleton data. It is to be noted that the one embodiment of the present disclosure is also applicable to any other motion capture technology and purpose. In addition, in the following description, the moving body is mainly assumed to be a person, by way of example. However, the embodiment of the present disclosure is similarly applicable to any other type of moving body such as an animal or a robot.

FIG. 1 is an explanatory diagram depicting the information processing system according to the one embodiment of the present disclosure. As depicted in FIG. 1, the information processing system according to the one embodiment of the present disclosure includes six sensor devices 10A to 10F, a distribution user terminal 20, a distribution server 30, and viewing user terminals 40. In FIG. 1, a user U1 is a distribution user who distributes an avatar image, while users U2 and U3 are viewing users who view the avatar image.

The distribution user terminal 20, the distribution server 30, and the viewing user terminals 40 are connected with one another via a network 12. The network 12 is a wired or wireless transmission path for transmitting information from a device connected to the network 12. For example, the network 12 may include a public network such as the internet, a telephone line network, or a satellite network, or various LANs (Local Area Networks) and WANs (Wide Area Networks) including the Ethernet (registered trademark). Further, the network 12 may include a dedicated line network such as IP-VPN (Internet Protocol-Virtual Private Network).

### (Sensor device 10)

Each of the sensor devices 10 includes an inertial sensor (including an IMU: Inertial Measurement Unit) such as an acceleration sensor for acquiring an acceleration and a gyro sensor (Angular Velocity Sensor) for acquiring an angular velocity. In addition, each of the sensor devices 10 may also include such a sensor as a geomagnetic sensor, an ultrasonic sensor, or an atmospheric pressure sensor.

It is desirable that the sensor devices 10A to 10F be worn on joint parts (e.g., the lower back, the head) which are the bases of the body, or around end parts (e.g., the wrists, the ankles, and the head) of the body. In the example of FIG. 1, the sensor device 10A is worn on the lower back of the distribution user U1. The sensor devices 10B and 10E are worn on the wrists. The sensor devices 10C and 10D are worn on the ankles. The sensor device 10F is worn on the head. It is to be noted that, hereinafter, body parts on which the sensor devices 10 are worn will also be referred to as wearing parts in some cases. In addition, the number of the sensor devices 10 and the positions of parts (positions of the wearing parts) on which the sensor devices 10 are worn are not limited to those in the example of FIG. 1. The number of the sensor devices 10 worn by the distribution user U1 may be greater or less than that in FIG. 1.

Such sensor devices 10 each acquire, as sensor data, the acceleration or angular velocity of the corresponding wearing part and transmit the sensor data to the distribution user terminal 20.

### (Distribution user terminal 20)

The distribution user terminal 20 is one example of an information processing device that is used by the distribution user U1. The distribution user terminal 20 receives sensor data from the sensor devices 10, and generates an avatar image of the distribution user U1 by using the received sensor data. The distribution user terminal 20 acquires wearing part information which indicates the positions and postures of the respective wearing parts, on the basis of the sensor data, and generates skeleton data that includes position information and posture information regarding the respective parts in the skeleton structure, on the basis of the wearing part information. The details of which will be explained later. Moreover, the distribution user terminal 20 generates an image of an avatar whose posture matches the posture indicated by the skeleton data. The distribution user terminal 20 transmits the generated avatar image to the distribution server 30, and requests distribution of the avatar image from the distribution server 30.

It is to be noted that the skeleton data is one example of the posture information that indicates a posture of the distribution user U1. In the present description, skeleton data acquired at one time point will also be referred to as a pose in some cases. In addition, time-series data regarding poses at n consecutive time points will also be referred to as a motion in some cases.

In addition, FIG. 1 depicts a notebook PC (personal computer) as the distribution user terminal 20. However, the distribution user terminal 20 may be any other type of information processing device such as a smartphone or a desktop PC.

### (Distribution server 30)

The distribution server 30 distributes the avatar image to the viewing user terminals 40 in response to the request made by the distribution user terminal 20. In FIG. 1, one distribution server 30 for providing a distribution service from a certain provider is depicted. However, there may be multiple providers for providing distribution services and multiple distribution servers 30. In this case, the distribution user terminal 20 can send a request for distribution of the avatar image, to the distribution server 30 that provides the distribution service designated by the distribution user U1.

### (Viewing user terminal 40)

The viewing user terminals 40 are information processing devices that are used by viewing users (e.g., the user U2 and the user U3 in FIG. 1). Each of the viewing user terminals 40 includes a display unit that displays various screens, an operation unit that detects an operation performed by the viewing user, and a control unit that controls the overall operation of the viewing user terminal 40. The viewing user terminal 40 makes a request for distribution of the avatar image of the distribution user U1 from the distribution server 30, according to an operation performed by the viewing user, for example, and displays the avatar image distributed by the distribution server 30.

FIG. 2 is an explanatory diagram depicting a specific example of an avatar image V which is displayed on the viewing user terminal 40. For example, as the avatar image V, an image of a two-dimensional character is displayed on the viewing user terminal 40, as depicted in FIG. 2. The posture of the distribution user U1 is reflected in the avatar image V. That is, the avatar image V varies in conjunction with the movement of the distribution user U1.

### (Background)

In some cases, however, skeleton data generated according to the motion capture technology is inexact. For example, in the sensor system, inexact skeleton data is generated in a case where a sensor device worn by a distribution user slips down or is displaced. In an optical system or the camera system, the accuracy of posture information can be degraded due to displacement of a camera or occurrence of a drift. As a result, there has been a concern that an avatar image may be generated on the basis of an inexact posture or motion in a case where the motion capture technology is applied to generation of avatar images, for example.

It is to be noted that calibration based on the motion capture technology may be performed in order to avoid distribution of an inexact avatar image. No sensor data is obtained during the calibration. Accordingly, an alternative image that matches the avatar world, an image including a message "please wait," or the like can be distributed until the calibration is completed. However, displaying such an image deprives the viewing user of the sense of immersion.

The present inventors have achieved the one embodiment of the present disclosure in view of the abovementioned circumstances. The information processing system according to the one embodiment of the present disclosure can deal with the generation of inexact skeleton data. The configuration and operation of the distribution user terminal 20 according to the one embodiment of the present disclosure as described above will sequentially be explained in detail below.

### <<2. Configuration of distribution user terminal>>

### <2-1. Overall configuration>

FIG. 3 is an explanatory diagram depicting the configuration of the distribution user terminal 20 according to the one embodiment of the present disclosure. As depicted in FIG. 3, the distribution user terminal 20 according to the one embodiment of the present disclosure includes an operation unit 216, a display unit 220, a communication unit 230, and a control unit 240.

The operation unit 216 is operated by a distribution user to input an instruction or information to the distribution user terminal 20. The display unit 220 displays many different display screens. For example, the display unit 220 displays a display screen that includes an avatar image generated by the control unit 240. The communication unit 230 communicates with the distribution server 30 over the network 12. For example, the communication unit 230 transmits the avatar image generated by the control unit 240, to the distribution server 30 over the network 12.

The control unit 240 controls the overall operation of the distribution user terminal 20. In particular, the control unit 240 according to the one embodiment of the present disclosure has a function of generating skeleton data regarding the distribution user on the basis of sensor data received from the sensor devices 10, and generating an image of an avatar whose posture matches the posture indicated by the skeleton data. In addition, the control unit 240 according to the one embodiment of the present disclosure further has a function of modifying the skeleton data. These functions of the control unit 240 are implemented by a base tool 250 and an application section 260 which are depicted in FIG. 3.

The base tool 250 has a function of generating skeleton data from sensor data, and a function of modifying skeleton data. In the present description, skeleton data generated from sensor data is referred to as raw skeleton data, while skeleton data generated by modifying raw skeleton data is referred to as modified skeleton data, in some cases. Further, raw skeleton data and modified skeleton data may simply be referred to as skeleton data without being distinguished from each other, in other cases. The base tool 250 supplies raw skeleton data or modified skeleton data to the application section 260.

The application section 260 implements a variety of functions in cooperation with the base tool 250. For example, the application section 260 generates an avatar image on the basis of skeleton data supplied from the base tool 250, and sends a request for distribution of the avatar image, to the distribution server 30. Here, the application section 260 may send a request for distribution of the avatar image and other content data in combination, to the distribution server 30. Examples of the other content data include background data and music data. It is to be noted that the developer of the base tool 250 and the developer of the application section 260 may be identical to each other or may be different from each other. Hereinafter, the functions of such a base tool 250 and application section 260 as described above will be explained in more detail.

### <2-2. Functions of base tool>

FIG. 4 is an explanatory diagram depicting functions of the base tool 250. As depicted in FIG. 4, the base tool 250 includes a sensor data acquisition section 251, a calibration section 252, a skeleton data generation section 253, a feature amount extraction section 254, an application interface 255, a basic use range storage section 256, a use range determination section 257, and a data modification section 258.

### (Sensor data acquisition section 251)

The sensor data acquisition section 251 acquires, from the sensor devices 10, sensor data that indicates the accelerations or angular velocities of the wearing parts.

### (Calibration section 252)

The calibration section 252 calibrates the sensor data acquired by the sensor data acquisition section 251. The calibration section 252 may perform calibration when use of the base tool 250 is started, or may perform calibration according to an operation performed by the distribution user.

### (Skeleton data generation section 253)

The skeleton data generation section 253 acquires wearing part information which indicates the positions and postures of the respective wearing parts, on the basis of the sensor data acquired by the sensor data acquisition section 251, and generates raw skeleton data that includes position information and posture information regarding the respective parts in the skeleton structure, on the basis of the wearing part information. Hereinafter, generation of raw skeleton data will more specifically be explained with reference to FIG. 5.

FIG. 5 is an explanatory diagram depicting a specific example of generating raw skeleton data. The skeleton data generation section 253 acquires, on the basis of the sensor data, wearing part information PD100 that includes position information and posture information regarding wearing parts P101 to P106 where the sensor devices 10A to 10F are respectively worn, as depicted on the left side in FIG. 5.

Moreover, as depicted on the right side in FIG. 5, the skeleton data generation section 253 acquires raw skeleton data SD100 that includes position information and posture information regarding the respective parts in the skeleton structure, on the basis of the wearing part information PD100 regarding the wearing parts P101 to P106. The raw skeleton data SD100 includes not only information regarding a wearing part SP101 corresponding to the wearing part P101 and information regarding a wearing part SP102 corresponding to the wearing part P102, but also information regarding a non-wearing part SP107.

It is to be noted that the raw skeleton data can include information regarding a bone (position information, posture information, etc.,) in addition to the information regarding the parts. For example, in the example depicted in FIG. 5, the raw skeleton data SD100 can include information regarding a bone SB101. The skeleton data generation section 253 is capable of identifying information regarding a bone between parts on the basis of position information and posture information regarding the parts in the skeleton structure.

### (Feature amount extraction section 254)

The feature amount extraction section 254 extracts a feature amount from the raw skeleton data (posture information regarding the distribution user) generated by the feature amount extraction section 254. For example, the feature amount extraction section 254 extracts a pose feature amount from a pose that is raw skeleton data acquired at one time point. The pose feature amount is a feature amount of the pose. If the feature amount extraction section 254 extracts pose feature amounts of similar poses, the pose feature amounts are positioned close to each other in a pose feature amount space. The feature amount extraction section 254 may extract a pose feature amount by using an identifier such as a DNN (deep neural network) that has subjected to learning so as to be able to extract such pose feature amounts.

In addition, the feature amount extraction section 254 extracts a motion feature amount from a motion that is time-series data regarding poses. The motion feature amount is a feature amount of the motion. If the feature amount extraction section 254 extracts motion feature amounts of similar motions, the motion feature amounts are positioned close to each other in a motion feature amount space. The feature amount extraction section 254 may extract a motion feature amount by using an identifier such as the DNN that has subjected to learning so as to be able to extract such motion feature amounts.

### (Application interface 255)

The application interface 255 is an interface to the application section 260. The application interface 255 may be formed as an API (Application Programming Interface). For example, the application interface 255 returns skeleton data regarding the distribution user to the application section 260 in response to a request sent from the application section 260. Specifically, in a case where modified skeleton data has been generated by the data modification section 258 which will be explained later, the application interface 255 may return the modified skeleton data to the application section 260. In a case where no modified skeleton data has been generated by the data modification section 258, the application interface 255 may return the raw skeleton data to the application section 260. In addition, the application interface 255 acquires information indicating an additional use range, which will be explained later, from the application section 260, and then passes the information indicating the additional use range to the use range determination section 257.

### (Basic use range storage section 256)

The basic use range storage section 256 stores information that indicates a basic use range which is one example of a definition range. The basic use range is a partial range in the pose feature amount space or the motion feature amount space. For example, the basic use range of a pose may include a feature amount of a pose that can be taken by a person in the pose feature amount space, but not a feature amount of a pose that is usually not anticipated to be taken by a person. Similarly, the basic use range of a motion may include a feature amount of a motion that can be made by a person in the motion feature amount space, but not a feature amount of a motion that is usually not anticipated to be made by a person. It is to be noted that the basic use range may indirectly be specified when information indicating the range of feature amounts that are not included within the basic use range is stored.

### (Use range determination section 257)

The use range determination section 257 determines whether or not the extracted feature amount which is a feature amount extracted by the feature amount extraction section 254 is included within the use range (definition range) in a feature amount space. The use range may be formed by the logical sum of the basic use range and the additional use range. For example, the use range determination section 257 determines whether or not the pose feature amount extracted by the feature amount extraction section 254 is included within the pose use range in the pose feature amount space. In addition, the use range determination section 257 determines whether or not the motion feature amount extracted by the feature amount extraction section 254 is included within the motion use range in the motion feature amount space.

### (Data modification section 258)

The data modification section 258 acquires, as a use feature amount, a feature amount that is included within the use range, when the use range determination section 257 determines that the feature amount extracted by the feature amount extraction section 254 is not included within the use range. Then, the data modification section 258 generates modified skeleton data that indicates a pose or a motion, by using the use feature amount. For example, the data modification section 258 acquires a use feature amount from among feature amounts included within the use range, according to the positional relation in the feature amount space (i.e., Euclidean distance) between the feature amount extracted by the feature amount extraction section 254 and each of the feature amounts included within the use range. More specifically, the data modification section 258 may acquire, as the use feature amount, a feature amount that is one of the feature amounts included within the use range and that is closest to the feature amount extracted by the feature amount extraction section 254. Now, a specific example of modified skeleton data which is generated by the data modification section 258 will be explained with reference to FIG. 6.

FIG. 6 is an explanatory diagram depicting a specific example of modified skeleton data generated by the data modification section 258. Raw skeleton data SD101 generated by the skeleton data generation section 253 is depicted on the left side in FIG. 6. In the raw skeleton data SD101, a left hand part is bent, but a left hand of a person does not usually have such a shape. Such inexact raw skeleton data SD101 can be generated in a case where the sensor device 10 is displaced or slips down.

In this case, the use range determination section 257 determines that the pose feature amount of the raw skeleton data SD101 is not included within the pose use range. Then, the data modification section 258 generates modified skeleton data MSD101 which is depicted on the right side in FIG. 6, for example. The modified skeleton data MSD101 is generated with the use of a feature amount that is included within the use range in the pose feature amount space. In the modified skeleton data MSD101, the bent left hand part has been modified into a linear shape.

The example in which the data modification section 258 generates the modified skeleton data for each pose has been given above. However, the data modification section 258 can also generate the modified skeleton data for each motion which is time-series data regarding multiple poses.

It is to be noted that, when generating the modified skeleton data, the data modification section 258 may use the feature amount (extracted feature amount) extracted from the raw skeleton data by the feature amount extraction section 254, in addition to a feature amount acquired from the use range. For example, the data modification section 258 may generate a combined feature amount by combining the extracted feature amount and the feature amount acquired from the use range, and may generate modified skeleton data that indicates a pose or a motion having the combined feature amount.

More specifically, the data modification section 258 may decide the combination ratio between the extracted feature amount and the feature amount acquired from the use range, according to the duration for which the extracted feature amount is determined not to be included within the use range. For example, the combination ratio of the feature amount acquired from the use range may be increased with the increase in the duration for which the extracted feature amount is determined not to be included within the use range. In this case, the initial modified skeleton data having the extracted feature amount determined not to be included within the use range is substantially identical to the raw skeleton data. As the duration for which the extracted feature amount is determined not to be included within the use range becomes longer, the difference between the modified skeleton data and the raw skeleton data becomes larger.

In contrast, in a case where the extracted feature amount is determined not to be included within the use range and is then determined to be included within the use range, the data modification section 258 still can continue generating modified skeleton data by using a combined feature amount. For example, the data modification section 258 may reduce the combination ratio of the feature amount acquired from the use range, as the time elapsed since the extracted feature amount is determined to be included within the use range becomes longer. In this case, with the increase in time elapsed since the extracted feature amount is determined to be included within the use range, the difference between the modified skeleton data and the raw skeleton data becomes smaller. When the time elapsed since the extracted feature amount is determined to be included within the use range reaches a predetermined time, the data modification section 258 may then stop generating the modified skeleton data.

### <2-3. Functions of application section>

The functions of the base tool 250 have been explained above. Next, functions of the application section 260 will be explained with reference to FIG. 7.

FIG. 7 is an explanatory diagram depicting functions of the application section 260. As depicted in FIG. 7, the application section 260 includes a base tool plug-in 261, an additional use range storage block 262, an additional use range registration block 263, a retargeting block 265, a display control block 267, and a distribution control block 268.

### (Base tool plug-in 261)

The base tool plug-in 261 is an interface to the base tool 250. The base tool plug-in 261 receives data from the base tool 250 and converts the data into a format that can be handled by the application section 260. For example, the base tool plug-in 261 receives, from the base tool 250, skeleton data which is raw skeleton data or modified skeleton data, for example.

### (Additional use range storage block 262)

The additional use range storage block 262 stores information that indicates an additional use range which is one example of the definition range. The additional use range is a partial range in the pose feature amount space or the motion feature amount space. The additional use range may include a feature amount of a pose or a motion that is suitable for a character being used for an avatar image, for example. It is to be noted that the additional use range may indirectly be specified when information indicating the range of feature amounts that are not included within the additional use range is stored.

### (Additional use range registration block 263)

The additional use range registration block 263 has a function of registering the additional use range into the additional use range storage block 262. The additional use range registration block 263 can register the additional use range into the additional use range storage block 262 in a variety of ways. Some exemplary methods for causing the additional use range registration block 263 to register an additional pose use range into the additional use range storage block 262 will be described below.

FIG. 8 is a flowchart indicating a first example of registering the additional use range. In the first registration example, first, the additional use range registration block 263 acquires a recorded motion (i.e., a set of poses acquired at sequential time points) (S302). Then, the feature amount extraction section 254 of the base tool 250 acquires the recorded motion from the additional use range registration block 263 via the application interface 255, and extracts a pose feature amount of each of the poses constituting the motion (S304).

Thereafter, the additional use range registration block 263 receives the pose feature amount of each of the poses constituting the motion from the base tool 250 via the base tool plug-in 261 and registers, as the additional pose use range, a range including the pose feature amount of each of the poses, into the additional use range storage block 262 (S306). It is to be noted that the additional pose use range may be a range where a Euclidean distance with respect to the pose feature amount of each of the poses is equal to or shorter than a predetermined length. Alternatively, the additional pose use ranges may be continuously located or may discretely be located in the feature amount space.

In a case of registering an additional motion use range, the feature amount extraction section 254 extracts a motion feature amount of a motion, and the additional use range registration block 263 receives the extracted motion feature amount from the base tool 250 via the base tool plug-in 261 and registers, as the additional motion use range, a range including the motion feature amount, into the additional use range storage block 262.

FIG. 9 is a flowchart indicating a second example of registering the additional use range. In the second registration example, first, a distribution user operates the operation unit 216 to designate a motion name, for example, walking, running, or the like (S312). It is to be noted that a database storing the correspondence between motions and motion names is assumed to be previously prepared in the base tool 250 or the application section 260.

The additional use range registration block 263 retrieves, from the database, a motion corresponding to the designated motion name (S314). Then, the feature amount extraction section 254 of the base tool 250 acquires the retrieved motion from the additional use range registration block 263 via the application interface 255, and extracts a pose feature amount of each of poses constituting the motion (S316).

Thereafter, the additional use range registration block 263 receives the pose feature amount of each of the poses constituting the motion, from the base tool 250 via the base tool plug-in 261, and registers, as the additional pose use range, a range including the pose feature amount of each of the poses, into the additional use range storage block 262 (S318).

In a case of registering the additional motion use range, the feature amount extraction section 254 extracts a motion feature amount of a motion, and the additional use range registration block 263 receives the extracted motion feature amount from the base tool 250 via the base tool plug-in 261 and registers, as the additional motion use range, a range including the motion feature amount, into the additional use range storage block 262.

FIG. 10 is a flowchart of a third example of registering the additional use range. In the third registration example, first, the display unit 220 displays a pose selection screen that includes multiple poses, and a distribution user operates the operation unit 216 to select two or more poses on the pose selection screen (S322). Here, a specific example of the pose selection screen will be explained with reference to FIG. 11.

FIG. 11 is an explanatory diagram depicting a specific example of the pose selection screen. The pose selection screen includes multiple pose indications 71A to 71C, selection buttons 72A to 72C corresponding to the pose indications 71A to 71C, respectively, and a new registration button 73, as depicted in FIG. 11. On such a pose selection screen, the distribution user selects, in order, the selection buttons 72 corresponding to the pose indications 71 representing the two or more desired poses, and presses the new registration button 73. It is to be noted that, in a case where a pose indication representing a desired pose is not included in the pose selection screen, the distribution user can also register a new pose by him- or herself.

Then, the additional use range registration block 263 derives a motion connecting the two or more poses selected by the distribution user, according to the selection order (S324). Subsequently, the feature amount extraction section 254 of the base tool 250 acquires the derived motion from the additional use range registration block 263 via the application interface 255, and extracts a pose feature amount of each of the poses constituting the motion (S326).

Thereafter, the additional use range registration block 263 receives the pose feature amount of each of the poses constituting the motion from the base tool 250 via the base tool plug-in 261, and registers, as the additional pose use range, a range including the pose feature amount of each of the poses, into the additional use range storage block 262 (S328).

In a case of registering the additional motion use range, the feature amount extraction section 254 extracts a motion feature amount of a motion, and the additional use range registration block 263 receives the extracted motion feature amount from the base tool 250 via the base tool plug-in 261 and registers, as the additional motion use range, a range including the motion feature amount, into the additional use range storage block 262.

### (Retargeting block 265)

The retargeting block 265 receives skeleton data regarding the distribution user from the base tool plug-in 261 and retargets the skeleton data, thereby generating an image of an avatar whose posture or motion matches the posture or motion indicated by the skeleton data.

### (Display control block 267)

The display control block 267 generates a variety of display screens and displays the generated display screen on the display unit 220. For example, the display control block 267 generates the abovementioned pose selection screen and displays the pose selection screen on the display unit 220. In addition, the display control block 267 generates an avatar display screen including an avatar image generated by the retargeting block 265 and displays the avatar display screen on the display unit 220.

### (Distribution control block 268)

The distribution control block 268 transmits distribution of the avatar image generated by the retargeting block 265, to the distribution server 30, and issues a request for distribution of the avatar image from the distribution server 30. In the following explanation, when the distribution of the avatar image is started, the display control block 267 generates a display screen designed for the distribution user and displays the display screen on the display unit 220. A specific example of a display screen generated by the display control block 267 will be described below.

FIG. 12 is an explanatory diagram depicting one example of a display screen designed for a distribution user. A distribution confirmation screen 81 is depicted on the left side in FIG. 12. The distribution confirmation screen 81 includes an avatar image V that is under distribution, a live indicator 811 that indicates that the avatar image V is being distributed on a real time basis, and an abnormality notification icon 813. Here, the avatar image V is generated from raw skeleton data, and the left leg of the avatar image V is bent to the outside.

The abnormality notification icon 813 indicates that a feature amount extracted from the raw skeleton data by the use range determination section 257 of the base tool 250 is determined not to be included within the use range. In a case where the pose feature amount of a pose having a left leg bent outward is not included within the use range, the abnormality notification icon 813 can be displayed, as depicted on the left side in FIG. 12.

When the distribution user selects the abnormality notification icon 813, the display control block 267 displays, on the display unit 220, the skeleton display screen 82 depicted on the right side in FIG. 12. The skeleton display screen 82 includes an indication 822 that indicates the raw skeleton data, an indication 823 that indicates an avatar image obtained when modified skeleton data is applied, and a modification button 824.

When the distribution user selects the modification button 824 on the skeleton display screen 82, the retargeting block 265 switches the retargeting object to the modified skeleton data. Accordingly, the skeleton display screen 83 including an indication 832 that indicates the modified skeleton data and an indication 833 that indicates an avatar image is displayed on the display unit 220, as depicted on the left side in FIG. 13.

Further, when the distribution user selects the indication 833 on the skeleton display screen 83, a distribution confirmation screen 84 depicted on the right side in FIG. 13 is displayed on the display unit 220. Since the retarget object is switched to the modified skeleton data, the left leg of the avatar image V is modified into a straight shape on the distribution confirmation screen 84. Further, the abnormality notification icon 813 in FIG. 12 has disappeared.

It is to be noted that the example in which the retarget object is switched to modified skeleton data according to the distribution user's operation has been explained above. However, the retargeting block 265 may automatically switch the retarget object to modified skeleton data in a case where the modified skeleton data has been generated. Alternatively, whether the switching to modified skeleton data is performed automatically or manually may be changed upon setting.

In addition, a calibration icon that is a guide to the calibration may be displayed as a predetermined notification on the display unit 220 by the display control block 267 when the number of times a feature amount extracted from raw skeleton data is determined not to be included within the use range or the frequency with which such a determination is made is greater than a threshold value.

FIG. 14 is an explanatory diagram depicting a specific example of a distribution confirmation screen 85 including the calibration icon. The distribution confirmation screen 85 includes the avatar image V that is generated from modified skeleton data, the live indicator 811, and a calibration icon 851, as depicted on the left side in FIG. 14.

When the distribution user selects the calibration icon 851 on the distribution confirmation screen 85, the display control block 267 generates a skeleton display screen 86, as depicted on the right side in FIG. 14. The skeleton display screen 86 includes an indication 862 that represents raw skeleton data, an indication 863 that represents the avatar image, and a calibration button 864. In the indication 862 representing the raw skeleton data, a part where the pose seems inexact, which is indicated by a dashed line, may be indicated in a specific color or thickness so as to be distinguished from the other parts. In addition, the colors or thicknesses of parts where the pose seems inexact may be changed according to the degrees of inexactness.

When the distribution user selects the calibration button 864 on the skeleton display screen 86, the calibration section 252 of the base tool 250 executes the calibration concerning the sensor device 10. After the calibration is executed, a distribution confirmation screen that includes the avatar image V but not the calibration icon 851 is displayed.

### <<3. Operation>>

The configuration of the information processing system according to the one embodiment of the present disclosure has been explained so far. Next, the operation of the information processing system according to the one embodiment of the present disclosure will be explained. It is to be noted that an example in which modified skeleton data is generated for each pose will mainly be described below. The operation which will be explained below is similarly applicable to a case where modified skeleton data is generated for each motion.

### <3-1. Operation of base tool 250>

FIG. 15 is a flowchart of the operation of the base tool 250. As depicted in FIG. 15, first, the skeleton data generation section 253 of the base tool 250 generates raw skeleton data of the current time on the basis of sensor data acquired by the sensor data acquisition section 251 (S404). Subsequently, the feature amount extraction section 254 extracts a pose feature amount from the raw skeleton data (S408).

Next, the use range determination section 257 determines whether or not the pose feature amount extracted by the feature amount extraction section 254 is within the use range in the feature amount space (S412). In a case where the extracted pose feature amount is within the use range in the feature amount space (Yes in S412), the application interface 255 supplies the raw skeleton data to the application section 260 (S416).

On the other hand, in a case where the extracted pose feature amount is not within the use range in the feature amount space (No in S412), the data modification section 258 acquires a pose feature amount that is within the use range (S420). Then, the data modification section 258 generates modified skeleton data by using the pose feature amount within the use range (S430). Here, the data modification section 258 may generate modified skeleton data that indicates a pose having the pose feature amount within the use range, or may generate a combined feature amount by combining the pose feature amount that is within the use range and the pose feature amount extracted from the raw skeleton data and then generate modified skeleton data that indicates a pose having the combined feature amount. The operation in the latter case will specifically be explained with reference to FIG. 16.

FIG. 16 is a flowchart of a specific example of a method of generating modified skeleton data. As indicated in FIG. 16, the data modification section 258 decides a combination ratio of the pose feature amount extracted from the raw skeleton data and the pose feature amount that is within the use range (S432). For example, the data modification section 258 may decide the combination ratio according to the duration for which the pose feature amount extracted from the raw skeleton data is determined not to be included within the use range. For example, the data modification section 258 may increase the combination ratio of the pose feature amount within the use range with the increase in the abovementioned duration.

Next, the data modification section 258 generates a combination feature amount by combining the two pose feature amounts in the decided combination ratio (S434). Further, the data modification section 258 generates modified skeleton data having the combination feature amount (S436).

Thereafter, the application interface 255 supplies the modified skeleton data to the application section 260 (S440), as indicated in FIG. 15. Then, the use range determination section 257 increments the counter value (S444). In a case where the counter value is greater than a threshold value (Yes in S448), the application interface 255 outputs, to the application section 260, a calibration recommendation notification that indicates that execution of the calibration is recommended (S452). After S452 or in a case where the counter value is less than the threshold value (No in S448), the processing of S404 and subsequence steps is repeated.

In the above example, the number of times the extracted pose feature amount is determined not to be included within the use range in the feature amount space is managed as a counter value. Alternatively, the frequency (frequency per unit time) with which the extracted pose feature amount is determined not to be included within the use range in the feature amount space may be managed such that the calibration recommendation notification is outputted when the frequency is greater than a threshold value.

### <3-2. Operation of application section 260>

FIG. 17 is a flowchart of the operation of the application section 260. As indicated in FIG. 17, first, skeleton data is supplied from the base tool 250 to the base tool plug-in 261 (S504). In a case where no modified skeleton data has been generated in the base tool 250, raw skeleton data is supplied. In a case where modified skeleton data has been generated in the base tool 250, the modified skeleton data is supplied. In a case where modified skeleton data has been generated in the base tool 250, not only the modified skeleton data but also the raw skeleton data may be supplied.

Then, the retargeting block 265 generates an avatar image by retargeting the skeleton data supplied from the base tool 250 (S508). In a case where no modified skeleton data has been generated, the retargeting block 265 retargets the raw skeleton data. In a case where modified skeleton data has been generated and supplied, the retargeting block 265 may switch the retarget object to the modified skeleton data automatically or according to the distribution user's operation.

The distribution control block 268 transmits the avatar image generated by the retargeting block 265 to the distribution server 30, and sends a request for distribution of the avatar image to the distribution server 30 (S512).

In a case where the calibration recommendation notification has been received from the base tool 250 (Yes in S516), the display control block 267 displays the distribution confirmation screen that includes the calibration icon, as previously explained with reference to FIG. 14 (S520). Then, when an instruction to execute the calibration is given through a distribution user's operation (Yes in S524), the application section 260 sends a request for execution of the calibration to the base tool 250 (S528). In a case where no calibration recommendation notification has been supplied (No in S516) or where no instruction to execute the calibration is given through a distribution user's operation (No in S524), or until an operation for ending the distribution is performed after S528, processing of S504 and subsequent steps is repeated (S548).

### <<4. Summery>>

According to the one embodiment of the present disclosure explained so far, a variety of effects are provided. For example, according to the one embodiment of the present disclosure, in a case where a feature amount extracted from raw skeleton data is not included within a use range in a feature amount space, modified skeleton data is generated by using a feature amount that is included within the use range. Accordingly, an exact and natural avatar image can be provided with the use of the modified skeleton data even in a case where the sensor device 10 worn by the distribution user slips down or is displaced and where raw skeleton data is generated on the basis of an inexact pose. Hence, in a case where live distribution of the avatar image is performed, the live distribution can be continued without giving an uncomfortable feeling to users. Also, in a case where the distribution user poses or moves in an ethically inexact manner, distribution of an inexact avatar image can be prevented with the use of the modified skeleton data.

Here, the data modification section 258 acquires a feature amount that is included within the use range, according to the position relation in the feature amount space between the feature amount extracted from the raw skeleton data and each of the feature amounts included within the definition range. For example, the data modification section 258 acquires a feature amount that is one of the feature amounts included within the use range and that is closest to the feature amount extracted from the raw skeleton data. With such a configuration, the data modification section 258 can generate modified skeleton data that includes a pose or motion approximated to a pose or motion intended by the distribution user.

In a case where the one embodiment of the present disclosure is applied to a situation in which a specific motion such as dancing is expected to be a main motion, the use range is narrowed in such a manner that motions other than the specific motion are not included within the use range. Accordingly, a motion that is deviated from the specific motion can be modified to a motion that is within the use range. As a result of this, the dancing can be expressed as if a high-definition motion capture system is used. Meanwhile, a recorded motion is not used in the present method. Hence, the distribution user is still allowed to make an ad-lib motion.

In addition, the data modification section 258 can generate a combined feature amount by combining a feature amount that is within the use range and a feature amount extracted from raw skeleton data. For example, the data modification section 258 decides the combination ratio of the pose feature amount within the use range according to the duration for which the pose feature amount extracted from the raw skeleton data is determined not to be included within the use range. With such a configuration, when the retarget object is switched from the raw skeleton data to the modified skeleton data, an uncomfortable feeling given to the viewing user can be lessened because the difference between the raw skeleton data and the modified skeleton data is made small.

Also in a case where the feature amount extracted from the raw skeleton data is determined not to be included within the use range and is then determined to be included within the use range, the data modification section 258 can similarly continue generating the modified skeleton data by using the combined feature amount. For example, the data modification section 258 may reduce the combination ratio of the feature amount within the use range with the increase in time elapsed since the feature amount extracted from the raw skeleton data is determined to be included within the use range. With such a configuration, when the retarget object is switched from the modified skeleton data to the raw skeleton data, an uncomfortable feeling given to the viewing user can be lessened because the difference between the raw skeleton data and the modified skeleton data is made small.

In addition, in a case where the number of times the feature amount extracted from the raw skeleton data is determined not to be included within the use range or the frequency with which such a determination is made is greater than a threshold value, the base tool 250 outputs the calibration recommendation notification. With such a configuration, it is expected that the feature amount extracted from the raw skeleton data is more likely to be included within the use range as a result of execution of the calibration. In this case, an avatar image is generated by using the raw skeleton data. Accordingly, an avatar image having a pose or a motion that is close to the intention of the distribution user can be generated.

In addition, the retargeting block 265 can also switch the retarget object according to a distribution user's operation. With such a configuration, the distribution user can have an option to generate an avatar image by using raw skeleton data, even in a case where the feature amount extracted from the raw skeleton data is not included within the use range.

In addition, the additional use range registration block 263 can define the additional use range, and a variety of definition methods can be used in a step for defining the additional use range. With such a configuration, the distribution user can easily define the additional use range according to an application.

### <<5. Modifications>>

The one embodiment of the present disclosure has been explained above. Some modifications of the abovementioned embodiment will be explained below. It is to be noted that the following modifications may separately be applied to the abovementioned embodiment or may be applied in combination to the abovementioned embodiment. In addition, each of the modifications may be applied in place of the configuration of the abovementioned embodiment or may be applied in addition to the configuration of the abovementioned embodiment.

### <4-1. First modification>

The example in which generation of modified skeleton data is omitted in a case where a feature amount extracted from raw skeleton data is included within the use range has been explained above. However, the data modification section 258 may generate modified skeleton data also in the case where a feature amount extracted from raw skeleton data is included within the use range. The present modification as a first modification will be explained with reference to FIG. 18.

FIG. 18 is a flowchart of the first modification of the operation of the base tool 250. Processing of steps S404 to S412 and S420 to S452 is the same as the processing previously explained with reference to FIG. 15.

In a case where the use range determination section 257 determines in S412 that the pose feature amount extracted by the feature amount extraction section 254 is within the use range in the feature amount space (Yes in S412), the data modification section 258 acquires a predicted feature amount from the use range (S413). The predicted feature amount is a feature amount of a predicted future pose or a predicted future motion of the distribution user. The data modification section 258 may input the current pose feature amount extracted by the feature amount extraction section 254, to an identifier such as a DNN, and acquire the predicted feature amount outputted from the identifier.

Subsequently, the data modification section 258 generates modified skeleton data by using the predicted feature amount (S414). The data modification section 258 may generate modified skeleton data that indicates a pose having the predicted feature amount, or may generate a combined feature amount by combining the predicated feature amount and the pose feature amount extracted from raw skeleton data and generate modified skeleton data that indicates a pose having the combined feature amount. Then, the application interface 255 supplies the modified skeleton data to the application section 260 (S415). Accordingly, in a case where the pose feature amount extracted from the raw skeleton data is within the use range, an avatar image can also be generated from the modified skeleton data that is generated as a result of the prediction.

Such a first modification as described above is useful for an application in which low delay is desired because the real-time property of an avatar image is important. In particular, in a case where the distribution user performs a dance which is assumed to have limited motions, for example, it is possible to predict modified skeleton data with high accuracy and reduce distribution delay on the basis of the modified skeleton data.

### <4-2. Second modification>

In the example described above, whether or not a feature amount of raw skeleton data regarding the whole body is within the use range is determined, and modified skeleton data regarding the whole body is generated. However, such determination and generation may be conducted for each part. The present modification as a second modification will be explained with reference to FIG. 19.

FIG. 19 is a flowchart of the second modification of the operation of the base tool 250. As depicted in FIG. 19, first, the skeleton data generation section 253 of the base tool 250 generates raw skeleton data of the current time for each part on the basis of sensor data acquired by the sensor data acquisition section 251 (S604). Then, the feature amount extraction section 254 extracts a pose feature amount from the raw skeleton data regarding each part (S608). It is to be noted that examples of the part includes a right arm, a left arm, a left leg, a right leg, and a trunk.

In the second modification, a use range is defined for each part. The use range determination section 257 determines whether or not pose feature amounts of all parts are within the corresponding use ranges defined for the parts (S612). In a case where the pose feature amounts of all parts are within the corresponding use ranges (Yes in S612), the application interface 255 supplies the raw skeleton data regarding the respective parts to the application section 260 (S616).

On the other hand, in a case where any of the parts has a pose feature amount outside the use range (No in S612), the data modification section 258 acquires, for an out-of-use range part which is the part having the pose feature amount outside the use range, a pose feature amount that is included within the use range of the out-of-use range part (S620). Subsequently, the data modification section 258 generates modified skeleton data regarding the out-of-use range part by using the pose feature amount included within the use range (S630).

Thereafter, the application interface 255 supplies, to the application section 260, the modified skeleton data regarding the out-of-use range part and raw skeleton data regarding a within-use range part which has a pose feature amount within the use range (S640). Then, the use range determination section 257 increments the counter value (S644). In a case where the counter value is greater than a threshold value (Yes in S648), the application interface 255 outputs, to the application section 260, the calibration recommendation notification that indicates that execution of the calibration is recommended (S652). After step S652 or in a case where the counter value is less than the threshold value (No in S648), processing of S604 and subsequence steps is repeated.

According to such a second modification as described above, determination as to the use range and generation of modified skeleton data can be conducted for each part. Accordingly, the accuracy of the determination and the generation of modified skeleton data can be increased.

### <4-3. Other modifications>

Moreover, many different modifications of the abovementioned embodiment can be made. For example, in the abovementioned embodiment, the basic use range is managed by the base tool 250, and the additional use range is managed by the application section 260. However, the management of the basic use range by the base tool 250 or the management of the additional use range by the application section 260 may be omitted. In this case, the use range may be the basic use range or the additional use range only.

In addition, the use range may be defined by a viewing user side. For example, the viewing user may operate the viewing user terminal 40 to select a pose or the like in order to allow an avatar image to take the selected pose or to prevent the avatar image from taking the selected pose, and a user use range that includes a feature amount of the selected pose may be defined. In this case, determination using the user use range and generation of modified skeleton data may be performed by the viewing user terminal 40 or may be performed by the distribution server 30 with the user use range being managed by the distribution server 30. With such a configuration, a pose or motion that the viewing user does not desire for an avatar image can be prevented from being displayed on the viewing user terminal 40.

In the example described above, the modified skeleton data is generated in a case where the feature amount extracted from the raw skeleton data is outside the use range. However, there are other ways to handle this case. For example, in the case where the feature amount extracted from the raw skeleton data is outside the use range, the base tool 250 may output a predetermined notification to the application section 260, and the application section 260 may perform predetermined image processing on the avatar image on the basis of the notification. The predetermined image processing may be mosaic processing for blurring the avatar image or particle processing, for example. With such a configuration, an uncomfortable feeling given to the viewing user can be lessened. Alternatively, the application section 260 may perform the abovementioned image processing on the avatar image on the basis of the notification outputted from the base tool 250, before display of the avatar image based on the modified skeleton data is started according to a distribution user's operation, for example.

In addition, the example in which one motion capture technology is used has been explained above. However, it is possible to use multiple motion capture technologies in parallel and obtain raw skeleton data according to each of the motion capture technologies. In this case, the use range determination section 257 determines whether or not a feature amount extracted from each piece of the raw skeleton data is included within the use range. The data modification section 258 generates modified skeleton data by using a feature amount that has been determined to be included within the use range, without using a feature amount that has been determined not to be included within the use range. In a case where it is determined that two or more feature amounts are included within the use range, the data modification section 258 may generate a combined feature amount by combining the two or more feature amounts, and generate modified skeleton data having the combined feature amount. Here, the data modification section 258 may perform combining in such a manner that the combination ratio of the feature amount of raw skeleton data obtained according to a higher-accuracy motion capture technology becomes higher. With such a configuration, distribution of an inexact avatar image can be prevented.

### <<6. Other configuration examples of information processing system>>

The configuration example in which the distribution user terminal 20 includes the base tool 250 and the application section 260 has been explained above as a first configuration example of the information processing system. However, other configuration examples of the information processing system according to the present disclosure are also conceivable. Hereinafter, such configuration examples of the information processing system will be explained.

### <5-1. Second configuration example>

FIG. 20 is an explanatory diagram depicting a second configuration example of the information processing system. The information processing system according to the second configuration example includes a distribution user terminal 20-2 and a processing terminal 50-2, as depicted in FIG. 20. The distribution user terminal 20-2 and the processing terminal 50-2 are connected to each other over the network 12. The distribution user terminal 20-2 includes the base tool 250 but not the application section 260. The application section 260 is mounted on the processing terminal 50-2.

In the second configuration example, the distribution user terminal 20-2 transmits raw skeleton data or modified skeleton data to the processing terminal 50-2. Then, the application section 260 of the processing terminal 50-2 generates an avatar image from the raw skeleton data or the modified skeleton data, and distributes the avatar image to the viewing user terminals 40 via the distribution server 30. In the second configuration example, the developer of the base tool 250 and the developer of the application section 260 may be identical to each other or may be different from each other.

### <5-2. Third configuration example>

FIG. 21 is an explanatory diagram depicting a third configuration example of the information processing system. The information processing system according to the third configuration example includes a distribution user terminal 20-3 and a processing terminal 50-3, as depicted in FIG. 21. The distribution user terminal 20-3 and the processing terminal 50-3 are connected to each other over the network 12. The distribution user terminal 20-3 includes the base tool 250 and an application section 260-3. The application section 260-3 does not include the retargeting block 265 and the distribution control block 268 which are included in the application section 260 having been explained with reference to FIG. 7. The processing terminal 50-3 includes the retargeting block 265 and the distribution control block 268, instead.

In the third configuration example, the distribution user terminal 20-3 transmits raw skeleton data or modified skeleton data to the processing terminal 50-3. Then, the retargeting block 265 of the processing terminal 50-3 generates an avatar image from the raw skeleton data or the modified skeleton data. The distribution control block 268 distributes the avatar image to the viewing user terminals 40 via the distribution server 30. In the third configuration example, the developer of the base tool 250, the developer of the application section 260-3, the developer of the retargeting block 265, and the developer of the distribution control block 268 may be identical to one another or may be different from one another.

### <5-3. Fourth configuration example>

FIG. 22 is an explanatory diagram depicting a fourth configuration example of the information processing system. The information processing system according to the fourth configuration example includes a distribution user terminal 20-4 and a processing terminal 50-4, as depicted in FIG. 22. The distribution user terminal 20-4 and the processing terminal 50-4 are connected to each other over the network 12. The distribution user terminal 20-4 includes the base tool 250. The processing terminal 50-4 includes an application section 260-4. The application section 260-4 does not include the function of the distribution control block 268. The processing terminal 50-4 additionally includes the function of the distribution control block 268.

In the fourth modification, the distribution user terminal 20-4 transmits raw skeleton data or modified skeleton data to the processing terminal 50-4. Then, the application section 260-4 of the processing terminal 50-4 generates an avatar image from the raw skeleton data or the modified skeleton data, and the distribution control block 268 distributes the avatar image to the viewing user terminals 40 via the distribution server 30. In the fourth configuration example, the developer of the base tool 250, the developer of the application section 260-4, and the developer of the distribution control block 268 may be identical to one another or may be different from one another.

### <5-4. Fifth configuration example>

FIG. 23 is an explanatory diagram depicting a fifth configuration example of the information processing system. The information processing system according to the fifth configuration example includes a distribution user terminal 20-5 and a processing terminal 50-5, as depicted in FIG. 23. The distribution user terminal 20-5 and the processing terminal 50-5 are connected to each other over the network 12. The distribution user terminal 20-5 includes the base tool 250. The processing terminal 50-5 includes an application section 260-5. The application section 260-5 does not include the function of the retargeting block 265 and the function of the distribution control block 268. The processing terminal 50-5 additionally includes the function of the retargeting block 265 and the function of the distribution control block 268.

In the fifth modification, the distribution user terminal 20-5 transmits raw skeleton data or modified skeleton data to the processing terminal 50-5. Then, the application section 260-5 supplies the raw skeleton data or the modified skeleton data to the retargeting block 265. The retargeting block 265 generates an avatar image from the raw skeleton data or the modified skeleton data. The distribution control block 268 distributes the avatar image to the viewing user terminals 40 via the distribution server 30. In the fifth configuration example, the developer of the base tool 250, the developer of the application section 260-5, the developer of the retargeting block 265, and the developer of the distribution control block 268 may be identical to one another or may be different from one another.

### <5-5. Sixth configuration example>

The example in which the functions of the operation unit 216, the display unit 220, the communication unit 230, and the control unit 240 are implemented by the distribution user terminal 20 of a PC type has mainly been explained above. However, these functions may be implemented by a mobile terminal such as a smartphone. Alternatively, these functions may be implemented by multiple mobile terminals in a distributed manner or in a distributed and overlapped manner. An example in which the abovementioned functions are performed by multiple mobile terminals in a distributed manner will be explained as a sixth configuration example with reference to FIG. 24.

FIG. 24 is an explanatory diagram depicting a sixth configuration example of the information processing system. The information processing system according to the sixth configuration example includes a first mobile terminal 61, a second mobile terminal 62, and a third mobile terminal 63, as depicted in FIG. 24.

The function of the control unit 240, i.e., the functions of the base tool 250 and the application section 260, is implemented by the first mobile terminal 61. In addition, the first mobile terminal 61 further includes a communication unit for communicating with the second mobile terminal 62 and the third mobile terminal 63. The first mobile terminal 61 generates an avatar image of a user U1 on the basis of sensor data acquired from the sensor devices 10, and transmits the avatar image to the second mobile terminal 62 and the third mobile terminal 63. It is to be noted that FIG. 24 depicts an example in which communication between the first mobile terminal 61, the second mobile terminal 62, and the third mobile terminal 63 is performed over the network 12. However, communication between the first mobile terminal 61, the second mobile terminal 62, and the third mobile terminal 63 may directly be performed without the network 12.

The second mobile terminal 62 implements the functions of the display unit 220 and the communication unit 230. The second mobile terminal 62 receives the avatar image from the first mobile terminal 61, and displays a display screen including the avatar image on the display unit 220. Accordingly, a user U4 who is using the second mobile terminal 62 can see the avatar image. The display screen displayed on that second mobile terminal 62 may be identical to the display screen having been explained with reference to FIGS. 12 to 14 and the like, or may not include the abnormality notification icon 813, the calibration icon 851, and the like.

The functions of the operation unit 216 and the communication unit 230 are implemented by the third mobile terminal 63. When a user U5 who is using the third mobile terminal 63 performs, on the operation unit 216, an operation for switching the retarget object to modified skeleton data or an operation for giving an instruction to execute the calibration, the third mobile terminal 63 transmits information indicating the operation to the first mobile terminal 61. For the operation as described above, the third mobile terminal 63 may have the function of the display unit 220 that displays a display screen including an avatar image.

It is to be noted that the function of the second mobile terminal 62 and the function of the third mobile terminal 63 may collectively be implemented by a single mobile terminal. Also, the second mobile terminal 62 and the third mobile terminal 63 each have the function of the application section 260. In this case, the first mobile terminal 61 may transmit skeleton data, in place of an avatar image, to the second mobile terminal 62 and the third mobile terminal 63, and the second mobile terminal 62 and the third mobile terminal 63 may each generate and display an avatar image from the skeleton data. In addition, some or all of the functions of the application section 260 may be implemented by each of the mobile terminals. For example, the function of the additional use range storage block 262 may be implemented by the first mobile terminal 61 and the third mobile terminal 63, the function of the additional use range registration block 263 may be implemented by the third mobile terminal 63, and the function of the display control block 267 may be implemented by the second mobile terminal 62 and the third mobile terminal 63.

The sixth configuration example of such an information processing system is assumed to be applied to many different use cases. As the use case of the sixth configuration example of the information processing system, cases where a user captures images outside, captures images while moving, and captures images under a particular environment are conceivable, for example. When the user captures images outside and captures images while moving, the necessity for preparing a power source, a machine transport facility, or the like is eliminated with the use of a mobile terminal. Accordingly, motion capturing and data processing can be performed while any heavy item is not being carried. In addition, in a case where the user U1 who is, for example, a performer carries the first mobile terminal 61 and where the first mobile terminal 61 transmits skeleton data or an avatar image to each of the second mobile terminals 62 that multiple users such as a producer and a director have, the skeleton data or the avatar image can immediately be checked under different environments.

In addition, when images are captured in a specific environment such as a concert venue or an open-air place, radio waves are likely to scatter. Therefore, it is difficult to receive sensor data from the sensor devices 10. In this regard, if a compact communication device such as the first mobile terminal 61 is worn by a performer, the distance between the first mobile terminal 61 and each sensor device 10 becomes short. Accordingly, the first mobile terminal 61 can receive the sensor data with high accuracy. The influence on a costume or the performance of the performer can be suppressed because a dedicated power source is not required. In addition, the second mobile terminal 62 or another display device can be placed in a point distant from the first mobile terminal 61.

It is to be noted that data regarding the posture of the user U1 can be obtained through a function included in the first mobile terminal 61. For example, if a camera in the first mobile terminal 61 is directed toward the user U1, the first mobile terminal 61 can obtain the direction of the face of the user U1. Further, the first mobile terminal 61 can capture the motion of the user U1 from images obtained by the camera.

Hence, in a case where a feature amount of raw skeleton data acquired from sensor data of the sensor devices 10 is not included within the use range, the first mobile terminal 61 may generate modified skeleton data by using data acquired by a function included in the first mobile terminal 61. For example, the first mobile terminal 61 may acquire, as the use feature amount, a feature amount that is one of the feature amounts included within the use range and that satisfies data acquired by a function included in the first mobile terminal 61, and may generate modified skeleton data by using the use feature amount.

In addition, in a case where the first mobile terminal 61 can infer the position and movement of the user U1 through a GNSS (Global Navigation Satellite System), SLAM (Simultaneous Localization and Mapping), or the like, modified skeleton data can be generated by using the inference result. For example, in a case where it is inferred that the user U1 slowly moves, the user U1 is considered to be walking. Thus, the first mobile terminal 61 can generate modified skeleton data having a walking posture.

### <<7. Hardware configuration>>

The embodiment according to the present disclosure has been explained above. The abovementioned information processing such as generation of skeleton data and extraction of a feature amount is performed through collaboration between software and hardware of the distribution user terminal 20. The hardware will be explained below.

FIG. 25 is a block diagram depicting a hardware configuration of the distribution user terminal 20. The distribution user terminal 20 includes a CPU (central processing unit) 201, a ROM (read only memory) 202, a RAM (random access memory) 203, and a host bus 204. The distribution user terminal 20 further includes a bridge 205, an external bus 206, an interface 207, an input device 208, an output device 210, a storage device (HDD) 211, a drive 212, and a communication device 215.

The CPU 201 functions as a computation processing device and a control device and controls the general operation of the distribution user terminal 20 according to various programs. The CPU 201 may also be a microprocessor. The ROM 202 stores, for example, programs and computation parameters to be used by the CPU 201. The RAM 203 temporally stores, for example, a program to be used for execution of the CPU 201 and a parameter that varies as appropriate during the execution. The CPU 201, the ROM 202, and the RAM 203 are mutually connected via the host bus 204 which includes a CPU bus or the like. Through collaboration of the CPU 201, the ROM 202, the RAM 203, and the software, the functions of the base tool 250 and the application section 260, which have been explained with reference to FIG. 3, can be implemented.

Via the bridge 205, the host bus 204 is connected to the external bus 206, which is a PCI (Peripheral Component Interconnect/Interface) bus, for example. It is to be noted that the host bus 204, the bridge 205, and the external bus 206 do not need to be formed into separate components, and the functions thereof may be implemented by a single bus.

The input device 208 includes, for example, input means for allowing a user to input information, such as a mouse, a keyboard, a touch panel, a button, a microphone, a switch, or a lever, and an input control circuit that generates an input signal on the basis of the input from the user and that outputs the input signal to the CPU 201. By operating the input device 208, a user of the distribution user terminal 20 can input various types of data or give an instruction for processing operation to the distribution user terminal 20.

The output device 210 includes a display device such as a liquid crystal display (LCD) device, an OLED (Organic Light Emitting Diode) device, and a lamp. Moreover, the output device 210 includes a sound output device such as a loudspeaker or headphones. The output device 210 outputs reproduced content, for example. Specifically, the display device displays various types of information such as reproduced image data in a text or image form, while the sound output device converts reproduced sound data, for example, to a sound and outputs the sound.

The storage device 211 is configured to store data. The storage device 211 is one example of the storage section of the distribution user terminal 20 according to the present embodiment. The storage device 211 may include a storage medium, a recorder that records data into the storage medium, a reader that reads out data from the storage medium, and a deleter that deletes data recorded in the storage medium. The storage device 211 includes an HDD (hard disk drive), for example. The storage device 211 drives a hard disk and stores a program or various types of data to be executed by the CPU 201.

The drive 212 is a reader/writer for a storage medium and is incorporated in the distribution user terminal 20 or is externally attached thereto. The drive 212 reads out information recorded in a removable storage medium 24 such as a magnetic disk, an optical disk, a magneto-optical disk, or a semiconductor memory when the removable storage medium 24 is attached thereto, and outputs the read information to the RAM 203. In addition, the drive 212 is also capable of writing information into the removable storage medium 24.

The communication device 215 is a communication interface including a communication device for establishing connection with the network 12, for example. In addition, the communication device 215 may be a wireless LAN (Local Area Network)-compatible communication device, an LTE (Long Term Evolution)-compatible communication device, or a wired communication device that performs wired communication.

It is to be noted that the explanation of the hardware configuration of the distribution user terminal 20 has been given with reference to FIG. 25, but an explanation of the hardware of the distribution server 30 and the hardware of the viewing user terminal 40 will be omitted because the configurations of the distribution server 30 and the viewing user terminal 40 can be substantially identical to that of the distribution user terminal 20.

### <<8. Supplemental remarks>>

The preferable embodiment of the present disclosure has been explained so far with reference the attached drawings. However, the present disclosure is not limited to the embodiment. It is clear that a person ordinarily skilled in the technical field of the present disclosure can conceive of various changes and modifications within the technical scope set forth in the claims. Such changes and modifications are also naturally considered to fall within the technical scope of the present disclosure.

For example, the functional blocks of the base tool 250, which have been explained with reference to FIG. 4, may be implemented by multiple terminals in a distributed manner. Similarly, the functional blocks of the application section 260, which have been explained with reference to FIG. 7, may also be implemented by multiple terminals in a distributed manner.

For example, the respective steps of the processing performed by the distribution user terminal 20 described in the present description are not necessarily executed in time series in the order described in the flowcharts. For example, the respective steps of the processing performed by the distribution user terminal 20 may be executed in an order different from that in the flowchart or may be executed in parallel.

In addition, it is also possible to create a computer program for causing the hardware including the CPU, the ROM, and the RAM in the distribution user terminal 20, for example, to implement functions that are equivalent to the functions of the abovementioned units of the distribution user terminal 20. Further, a storage medium having the computer program stored therein is also provided.

Moreover, the effects described herein are merely explanatory or illustrative, and effects are not limited to the abovementioned effects. That is, the technology according to the present disclosure can provide, in addition to or in place of the abovementioned effects, any other effect that is obvious from the present description to a person skilled in the art.

Note that the technical scope of the present disclosure also covers the following configurations.
(1) An information processing method including:
   acquiring posture information that indicates a posture of a moving body;
   extracting a feature amount from the posture information acquired at one time point or at multiple time points;
   determining whether or not the extracted feature amount is included within a definition range in a feature amount space; and
   acquiring, as a use feature amount, a feature amount that is included within the definition range, when the extracted feature amount is determined not to be included within the definition range, and generating data that indicates a posture or a motion, by using the use feature amount.
(2) The information processing method according to (1) above, in which
   the generating the data includes acquiring the use feature amount from among feature amounts included within the definition range, according to a positional relation in the feature amount space between the extracted feature amount and each of the feature amounts included within the definition range.
(3) The information processing method according to (2) above, in which
   the acquiring the use feature amount from among the feature amounts included within the definition range includes acquiring, as the use feature amount, a feature amount that is one of the feature amounts included within the definition range and that is closest to the extracted feature amount in the feature amount space.
(4) The information processing method according to any one of (1) to (3) above, in which
   the generating the data includes generating a combined feature amount by combining the extracted feature amount and the use feature amount, and generating data that indicates a posture or a motion having the combined feature amount.
(5) The information processing method according to (4) above, in which
   the generating the combined feature amount includes combining the extracted feature amount and the use feature amount in a ratio that corresponds to duration for which the extracted feature amount is determined not to be included within the definition range.
(6) The information processing method according to (5) above, in which
   the generating the combined feature amount includes increasing a ratio of combining the use feature amount with an increase in the duration.
(7) The information processing method according to any one of (4) to (6) above, in which,
   in a case where the extracted feature amount is determined not to be included within the definition range and is then determined to be included within the definition range, the generating the combined feature amount includes reducing a ratio of combining the use feature amount with an increase in time elapsed since the extracted feature amount is determined to be included within the definition range.
(8) The information processing method according to any one of (1) to (7) above, further including:
   controlling output of a predetermined notification to a user when the number of times or a frequency with which the extracted feature amount is determined not to be included within the definition range is higher than a threshold value.
(9) The information processing method according to (8) above, in which
   the predetermined notification includes a guide to calibration of a sensor for acquiring the posture information.
(10) The information processing method according to any one of (1) to (9) above, further including:
   acquiring a predicted feature amount that indicates a predicted future posture or motion of the moving body, from among feature amounts included within the definition range, when the extracted feature amount is determined to be included within the definition range, and generating data that indicates a posture or a motion, by using the predicted feature amount.
(11) The information processing method according to any one of (1) to (10) above, in which
   the generating the data includes outputting, to a user, a notification indicating that the extracted feature amount is not included within the definition range, when the extracted feature amount is determined not to be included within the definition range, and generating data that indicates a posture or a motion, by using the use feature amount, when the user has performed an operation to give an instruction to adjust the posture or the motion.
(12) The information processing method according to any one of (1) to (11) above, in which
   the information processing method is performed for each of one or two or more of multiple parts constituting the moving body.
(13) The information processing method according to any one of (1) to (12) above, further including:
   a definition step of defining the definition range, in which
   the definition step includes
      acquiring the posture information that indicates the posture of the moving body,
      extracting the feature amount from the posture information acquired at one time point or at multiple time points, and
      defining the definition range in such a manner that the extracted feature amount is included within the definition range.
(14) The information processing method according to any one of (1) to (12) above, further including:
   a definition step of defining the definition range, in which
   the definition step includes
      acquiring the posture information that indicates the posture of the moving body,
      extracting a feature amount of a motion obtained by connecting pieces of the posture information acquired at multiple time points, or a feature amount of each of postures constituting the motion, and
      defining the definition range in such a manner that the extracted feature amount is included within the definition range.
(15) The information processing method according to any one of (1) to (12) above, further including:
   a definition step of defining the definition range, in which
   the definition step includes
      defining the definition range in such a manner that the definition range includes a feature amount of a posture or a motion that is designated by a user from among previously registered postures or motions.
(16) The information processing method according to any one of (1) to (15) above, further including:
   generating an image of an avatar whose posture or motion matches the posture or the motion indicated by the generated data.
(17) The information processing method according to (16) above, further including:
   distributing the image of the avatar over a network.
(18) The information processing method according to (1) above, in which
   the acquiring the posture information includes acquiring, by using different motion capture technologies, the posture information according to each of the motion capture technologies, and
   the generating the data includes generating the data by using, when the extracted feature amount of the posture information acquired according to any one of the motion capture technologies is determined not to be included within the definition range, an extracted feature amount that is acquired according to another one of the motion capture technologies and that is determined to be included within the definition range, as the use feature amount.
(19) An information processing device including:
   a posture information acquisition section that acquires posture information that indicates a posture of a moving body;
   a feature amount extraction section that extracts a feature amount from the posture information acquired at one time point or at multiple time points;
   a determination section that determines whether or not an extracted feature amount that is the feature amount extracted by the feature amount extraction section is included within a definition range in a feature amount space; and
   a data generation section that generates data that indicates a posture or a motion having a feature amount included within the definition range, when the determination section determines that the extracted feature amount is not included within the definition range.
(20) A program for causing a computer to function as:
   a posture information acquisition section that acquires posture information that indicates a posture of a moving body;
   a feature amount extraction section that extracts a feature amount from the posture information acquired at one time point or at multiple time points;
   a determination section that determines whether or not an extracted feature amount that is the feature amount extracted by the feature amount extraction section is included within a definition range in a feature amount space; and
   a data generation section that generates data that indicates a posture or a motion having a feature amount included within the definition range, when the determination section determines that the extracted feature amount is not included within the definition range.
(21) An information processing device including:
   a display control section that generates an image of an avatar whose posture or motion matches a posture or a motion indicated by data that is generated from a feature amount included within a definition range in a feature amount space, in a case where an extracted feature amount that is a feature amount obtained by extracting a feature amount from posture information regarding a moving body acquired at one time point or multiple time points is not included within the definition range.

### [Reference Signs List]

10: Sensor device
20: Distribution user terminal
216: Operation unit
220: Display unit
230: Communication unit
240: Control unit
250: Base tool
251: Sensor data acquisition section
252: Calibration section
253: Skeleton data generation section
254: Feature amount extraction section
255: Application interface
256: Basic use range storage section
257: Use range determination section
258: Data modification section
260: Application section
261: Base tool plug-in
262: Additional use range storage block
263: Additional use range registration block
265: Retargeting block
267: Display control block
268: Distribution control block
30: Distribution server
40: Viewing user terminal
50: Processing terminal

## Claims

1. An information processing method comprising:
acquiring posture information that indicates a posture of a moving body;
extracting a feature amount from the posture information acquired at one time point or at multiple time points;
determining whether or not the extracted feature amount is included within a definition range in a feature amount space; and
acquiring, as a use feature amount, a feature amount that is included within the definition range, when the extracted feature amount is determined not to be included within the definition range, and generating data that indicates a posture or a motion, by using the use feature amount.

2. The information processing method according to claim 1, wherein
the generating the data includes acquiring the use feature amount from among feature amounts included within the definition range, according to a positional relation in the feature amount space between the extracted feature amount and each of the feature amounts included within the definition range.

3. The information processing method according to claim 2, wherein
the acquiring the use feature amount from among the feature amounts included within the definition range includes acquiring, as the use feature amount, a feature amount that is one of the feature amounts included within the definition range and that is closest to the extracted feature amount in the feature amount space.

4. The information processing method according to claim 1, wherein
the generating the data includes generating a combined feature amount by combining the extracted feature amount and the use feature amount, and generating data that indicates a posture or a motion having the combined feature amount.

5. The information processing method according to claim 4, wherein
the generating the combined feature amount includes combining the extracted feature amount and the use feature amount in a ratio that corresponds to duration for which the extracted feature amount is determined not to be included within the definition range.

6. The information processing method according to claim 5, wherein
the generating the combined feature amount includes increasing a ratio of combining the use feature amount with an increase in the duration.

7. The information processing method according to claim 4, wherein,
in a case where the extracted feature amount is determined not to be included within the definition range and is then determined to be included within the definition range, the generating the combined feature amount includes reducing a ratio of combining the use feature amount with an increase in time elapsed since the extracted feature amount is determined to be included within the definition range.

8. The information processing method according to claim 1, further comprising:
controlling output of a predetermined notification to a user when the number of times or a frequency with which the extracted feature amount is determined not to be included within the definition range is higher than a threshold value.

9. The information processing method according to claim 8, wherein
the predetermined notification includes a guide to calibration of a sensor for acquiring the posture information.

10. The information processing method according to claim 1, further comprising:
acquiring a predicted feature amount that indicates a predicted future posture or motion of the moving body, from among feature amounts included within the definition range, when the extracted feature amount is determined to be included within the definition range, and generating data that indicates a posture or a motion, by using the predicted feature amount.

11. The information processing method according to claim 1, wherein
the generating the data includes outputting, to a user, a notification indicating that the extracted feature amount is not included within the definition range, when the extracted feature amount is determined not to be included within the definition range, and generating data that indicates a posture or a motion, by using the use feature amount, when the user has performed an operation to give an instruction to adjust the posture or the motion.

12. The information processing method according to claim 1, wherein
the information processing method is performed for each of one or two or more of multiple parts constituting the moving body.

13. The information processing method according to claim 1, further comprising:
a definition step of defining the definition range, wherein
the definition step includes
acquiring the posture information that indicates the posture of the moving body,
extracting the feature amount from the posture information acquired at one time point or at multiple time points, and
defining the definition range in such a manner that the extracted feature amount is included within the definition range.

14. The information processing method according to claim 1, further comprising:
a definition step of defining the definition range, wherein
the definition step includes
acquiring the posture information that indicates the posture of the moving body,
extracting a feature amount of a motion obtained by connecting pieces of the posture information acquired at multiple time points, or a feature amount of each of postures constituting the motion, and
defining the definition range in such a manner that the extracted feature amount is included within the definition range.

15. The information processing method according to claim 1, further comprising:
a definition step of defining the definition range, wherein
the definition step includes
defining the definition range in such a manner that the definition range includes a feature amount of a posture or a motion that is designated by a user from among previously registered postures or motions.

16. The information processing method according to claim 1, further comprising:
generating an image of an avatar whose posture or motion matches the posture or the motion indicated by the generated data.

17. The information processing method according to claim 16, further comprising:
distributing the image of the avatar over a network.

18. The information processing method according to claim 1, wherein
the acquiring the posture information includes acquiring, by using different motion capture technologies, the posture information according to each of the motion capture technologies, and
the generating the data includes generating the data by using, when the extracted feature amount of the posture information acquired according to any one of the motion capture technologies is determined not to be included within the definition range, an extracted feature amount that is acquired according to another one of the motion capture technologies and that is determined to be included within the definition range, as the use feature amount.

19. An information processing device comprising:
a posture information acquisition section that acquires posture information that indicates a posture of a moving body;
a feature amount extraction section that extracts a feature amount from the posture information acquired at one time point or at multiple time points;
a determination section that determines whether or not an extracted feature amount that is the feature amount extracted by the feature amount extraction section is included within a definition range in a feature amount space; and
a data generation section that generates data that indicates a posture or a motion having a feature amount included within the definition range, when the determination section determines that the extracted feature amount is not included within the definition range.

20. A program for causing a computer to function as:
a posture information acquisition section that acquires posture information that indicates a posture of a moving body;
a feature amount extraction section that extracts a feature amount from the posture information acquired at one time point or at multiple time points;
a determination section that determines whether or not an extracted feature amount that is the feature amount extracted by the feature amount extraction section is included within a definition range in a feature amount space; and
a data generation section that generates data that indicates a posture or a motion having a feature amount included within the definition range, when the determination section determines that the extracted feature amount is not included within the definition range.
